(19) Europäisches Patentamt / European Patent Office / Office européen des brevets

(11) **EP 2 516 623 B1**

# (12) FASCICULE DE BREVET EUROPEEN

(45) Date de publication et mention de la délivrance du brevet:
**05.10.2016  Bulletin 2016/40**

(21) Numéro de dépôt: **10810863.0**

(22) Date de dépôt: **21.12.2010**

(51) Int Cl.:
**C12N 5/071** (2010.01)    **C12N 7/00** (2006.01)

(86) Numéro de dépôt international:
**PCT/FR2010/052850**

(87) Numéro de publication internationale:
**WO 2011/077035 (30.06.2011 Gazette 2011/26)**

(54) **PROCEDE DE CULTURE DE CELLULES ADHERENTES**

METHODE ZUR KULTIVIERUNG VON ADHÄRENTEN ZELLEN ZU MIKROCARRIER

METHOD FOR CULTURING ADHERENT CELLS TO MICROCARRIERS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.12.2009  FR 0959472**
                 **11.03.2010  FR 1051754**

(43) Date de publication de la demande:
**31.10.2012  Bulletin 2012/44**

(73) Titulaire: **Sanofi Pasteur**
**69367 Lyon Cedex 07 (FR)**

(72) Inventeurs:
- **CALVOSA, Eric**
  **69610 Haute Rivoire (FR)**
- **SEVE, Nicolas**
  **69770 Chambost Longessaigne (FR)**

(74) Mandataire: **Ayroles, Marie-Pauline et al**
**Sanofi Pasteur**
**Direction Propriété Intellectuelle**
**2, avenue Pont Pasteur**
**69367 Lyon cedex 07 (FR)**

(56) Documents cités:
- XIAO C ET AL: "High density cultivation of genetically-engineered CHO cell lines with microcarrier culture systems.", CHINESE MEDICAL SCIENCES JOURNAL = CHUNG-KUO I HSÜEH K'O HSÜEH TSA CHIH / CHINESE ACADEMY OF MEDICAL SCIENCES JUN 1994 LNKD- PUBMED:8000063, vol. 9, no. 2, juin 1994 (1994-06), pages 71-74, XP009134567, ISSN: 1001-9294
- OHLSON S ET AL: "Bead-to-bead transfer of Chinese hamster ovary cells using macroporous microcarriers", CYTOTECHNOLOGY, vol. 14, no. 1, 1994, pages 67-80, XP009134574, ISSN: 0920-9069
- YUSHU WANG ET AL: "Bead-to-bead transfer of Vero cells in microcarrier culture", CYTOTECHNOLOGY, KLUWER ACADEMIC PUBLISHERS, DO LNKD- DOI:10.1023/A:1008079013375, vol. 31, no. 3, 1 novembre 1999 (1999-11-01), pages 221-224, XP019236658, ISSN: 1573-0778
- LANDAUER K ET AL: "Detachment factors for enhanced carrier to carrier transfer of CHO cell lines on macroporous microcarriers", CYTOTECHNOLOGY, KLUWER ACADEMIC PUBLISHERS, DO, vol. 39, no. 1, 1 mai 2002 (2002-05-01), pages 37-45, XP019236731, ISSN: 1573-0778, DOI: DOI:10.1023/A:1022455525323
- ROUROU S ET AL: "A novel animal-component-free medium for rabies virus production in Vero cells grown on Cytodex 1 microcarriers in a stirred bioreactor", APPLIED MICROBIOLOGY AND BIOTECHNOLOGY, SPRINGER VERLAG, BERLIN, DE, vol. 85, no. 1, 1 novembre 2009 (2009-11-01), pages 53-63, XP002599123, ISSN: 0175-7598, DOI: DOI:10.1007/S00253-009-2064-Y [extrait le 2009-06-12]

## Description

L'invention a pour objet un procédé de production de cellules adhérentes selon lequel on introduit des cellules adhérentes dans un récipient de culture qui contient des microporteurs dans un milieu de culture et on effectue plusieurs passages cellulaires successifs dans ce même récipient en utilisant à chaque fois tout ou partie de la population cellulaire du passage cellulaire précédent pour réaliser le passage cellulaire suivant. Elle concerne également la mise en oeuvre de ce procédé pour la production d'agents biologiques, servant notamment à la préparation de vaccins ou de médicaments.

Dans les années 80, le développement de la technologie de culture cellulaire sur microporteurs a facilité la production à grande échelle de cellules adhérentes et par voie de conséquence la production d'agents biologiques. La production de cellules adhérentes destinées à la production d'agents biologiques pour un usage pharmaceutique doit respecter néanmoins un certain nombre de prescriptions réglementaires notamment : celle de proscrire l'utilisation de cellules adhérentes au-delà d'un certain nombre de «passages cellulaires» car il existe un risque de transformation morphologique et/ou biologique des cellules. C'est le cas notamment des cellules de la lignée Vero.

US 4,664,912 décrit un procédé utilisé à l'échelle industrielle pour produire un lot de cellules adhérentes à partir d'une semence de cellules provenant d'une banque cellulaire de travail. Il repose sur une succession de passages cellulaires qui ont lieu à chaque fois dans des bioréacteurs différents dont les volumes utiles augmentent au cours des passages cellulaires successifs. Cela permet d'augmenter à chaque fois la quantité de microporteurs tout en maintenant une concentration optimale en microporteurs dans le milieu de culture qui est comprise généralement entre 1 et 5g/l. La biomasse cellulaire augmente ainsi au cours des passages cellulaires successifs jusqu'à obtenir le lot industriel de cellules désiré. Le transfert des cellules d'un bioréacteur à un autre bioréacteur est réalisé après avoir décroché les cellules adhérentes des microporteurs au moyen d'un traitement à la trypsine puis en bloquant l'action de l'enzyme en introduisant des protéines sériques ou du sérum dans le milieu pour préserver autant que possible l'intégrité des cellules. La suspension de cellules obtenue est alors transférée (en présence ou en l'absence des microporteurs usagés) dans un bioréacteur plus grand qui contient une quantité plus importante de nouveaux microporteurs. Cette méthode de production industrielle de cellules adhérentes requiert cependant l'utilisation et la manipulation d'un matériel important ce qui a une incidence sur les coûts de production des agents biologiques.

Pour diminuer les coûts de production de cellules adhérentes destinées à la production d'agents biologiques, EP 1060241 propose une méthode de production plus rapide qui ne nécessite plus le besoin de repartir d'une semence de cellules provenant d'une banque de travail à chaque fois que l'on veut produire un lot industriel de cellules. La méthode consiste à transférer, après chaque passage cellulaire, la plus grande partie des cellules (80 à 90% de la biomasse cellulaire) dans un ou plusieurs autres bioréacteurs pour poursuivre l'amplification de la biomasse cellulaire et constituer un lot de production industriel de cellules tandis que les 10 à 20 % de cellules restantes sont entretenues pour maintenir un stock « de cellules nourricières » à partir desquelles on peut produire de nouveaux lots de cellules. Cette méthode a néanmoins les inconvénients suivants :

- les lots de cellules produits présentent une certaine hétérogénéité dans la mesure où ils n'ont pas tous le même nombre de passages cellulaires.
- Le maintien d'un stock de cellules « nourricières » en culture lors de chaque opération de transfert entraine inévitablement un « vieillissement » des cellules qui est directement lié au nombre de passages cellulaires effectués et ne peut donc être utilisé que pendant une période de temps limité pour les raisons règlementaires déjà évoquées.

Pour s'affranchir de l'utilisation d'enzymes protéolytiques telles que la trypsine qui est néfaste à l'intégrité des cellules, Ohlson et coll dans Cytotechnology (1994), vol 14, pages 67-80 décrit une technologie de transfert des cellules adhérentes de microporteur à microporteur (bead to bead transfer) en l'absence de tout traitement enzymatique. Le contact étroit entre des microporteurs recouverts de cellules adhérentes et des microporteurs nus favorise le transfert des cellules sur les microporteurs nus à partir desquels les cellules peuvent s'amplifier. Pour augmenter la croissance cellulaire on rajoute donc des microporteurs nus à un milieu de culture qui contient des microporteurs recouverts de cellules adhérentes et en agitant de façon intermittente le milieu de culture pour favoriser le contact entre microporteurs Néanmoins la population de cellules qui est produite est « désynchronisée » puisque les cellules sont à des stades variés du cycle cellulaire ce qui peut être un inconvénient majeur pour produire des agents biologiques.

Xiao et al. (Chinese Medical Sciences Journal, 1994, 9 : 71-74) and Wang et al. (Cytotechnology, 1994, 31: 221-224) décrivent des procédés de production de cellules adhérentes à des microporteurs dans un seul récipient de culture selon lesquels est ajouté à chaque passage une population de microporteurs nus à une population de microporteurs recouverts de cellules adhérentes pour augmenter la production de cellules. Le contact entre les microporteurs nus et les microporteurs recouverts de cellules adhérentes permet le transfert des cellules sur les microporteurs nus à partir desquels les cellules peuvent s'amplifier. L'amplification des cellules se fait donc au moyen du transfert de cellules adhérentes de microporteur à microporteur (« bead-to-bead transfer ») en l'absence de tout traitement enzymatique.

Il existe toujours le besoin d'optimiser les méthodes de production à grande échelle de cellules adhérentes ainsi que la production d'agents biologiques qui en dérivent de façon à diminuer les coûts de production.

A cet effet, la présente invention a pour objet :

Un procédé de production de cellules adhérentes selon lequel :

a. on introduit des cellules adhérentes dans un récipient de culture qui contient des microporteurs dans un milieu de culture :

b. on amplifie les cellules en effectuant plusieurs passages cellulaires successifs dans ce même récipient de culture dans lequel chaque passage cellulaire ultérieur au premier passage cellulaire est réalisé :

i. en utilisant tout ou partie de la population cellulaire qui a été obtenue lors du passage cellulaire précédent après avoir soumis la population cellulaire à un traitement enzymatique pour détacher les cellules des microporteurs; et

ii. en introduisant du milieu de culture et une quantité croissante de microporteurs; et

c. On récolte la population cellulaire produite lors du dernier passage cellulaire après avoir, de façon optionnelle, soumis la dite population cellulaire à un traitement enzymatique pour détacher les cellules des microporteurs.

Elle a également pour objet :

Un procédé de production d'un agent biologique produit par des cellules adhérentes selon lequel :

a. on introduit des cellules adhérentes dans un récipient de culture qui contient des microporteurs dans un milieu de culture ;

b. on amplifie les cellules en effectuant plusieurs passages cellulaires successifs dans ce même récipient de culture dans lequel chaque passage cellulaire ultérieur au premier passage cellulaire est réalisé:

i. en utilisant tout ou partie de la population cellulaire qui a été obtenue lors du passage cellulaire précédent après avoir soumis la dite population cellulaire à un traitement enzymatique pour détacher les cellules des microporteurs, et

ii. en introduisant du milieu de culture et une quantité croissante de microporteurs;

c. on traite la population cellulaire produite lors du dernier passage cellulaire de manière à ce qu'elle produise l'agent biologique, le dit traitement étant réalisé dans le même récipient de culture que celui qui a été utilisé pour amplifier les cellules et ;

d. on récolte l'agent biologique.

Selon un aspect du procédé production de l'agent biologique, l'agent biologique est un agent infectieux et le traitement de la population cellulaire est effectué en infectant la population cellulaire produite lors du dernier passage cellulaire avec ledit agent infectieux dans un milieu d'infection.

Selon un aspect particulier, l'agent infectieux est le virus rabique et le milieu d'infection est un milieu d'infection virale dépourvu de tout produit d'origine animale.

Généralement le nombre de passages cellulaires effectués dans le même récipient de culture est 2, 3 ou 4.

La concentration en microporteurs dans le milieu de culture pendant le premier passage cellulaire est généralement < 1 g/l, et de façon préférée ≤ 0,5 g/l.

De façon très préférée, le traitement enzymatique emploie une solution contenant une enzyme protéolytique, telle que la trypsine.

Selon un autre aspect du procédé selon l'invention, chaque passage cellulaire ultérieur au premier passage cellulaire est réalisé en augmentant le volume du milieu de culture.

De façon préférée, le premier passage cellulaire est effectué dans un volume de milieu de culture qui est entre le 1/5 et la moitié du volume utile du récipient de culture.

Dans un autre mode du procédé selon l'invention, le milieu de culture est dépourvu de sérum d'origine animale

De façon préférée, le milieu de culture est dépourvu de produit d'origine animale.

Selon un autre aspect, la concentration en protéines dans le milieu de culture est ≤ 15 mg/l.

Selon encore un autre aspect, le milieu de culture contient en outre un agent protecteur cellulaire.

De façon préférée, l'agent protecteur cellulaire est une polyvinyl pyrrolidone ou un poloxamère.

Selon encore un autre mode de réalisation du procédé selon l'invention, le récipient de culture est un bioréacteur ayant un volume utile compris entre 3 et 3000 litres, de façon préférée entre 20 et 1000 litres et de façon particulièrement préférée entre 20 et 500 litres.

Dans encore un autre mode, le récipient de culture est un bioréacteur à usage unique.

Dans un aspect particulier du procédé selon l'invention, les cellules adhérentes sont des cellules Vero.

De façon générale, la population cellulaire qui est récoltée contient une quantité de cellules qui est au moins 60 fois plus importante que la quantité de cellules qui a été initialement introduite dans le récipient de culture.

Dans un autre aspect, l'invention concerne un procédé de production de cellules adhérentes selon lequel :

a. on décongèle un stock de cellules adhérentes, puis

b. on soumet les cellules adhérentes congelées à l'un des modes de réalisation du procédé de l'invention.

Dans encore un autre aspect, l'invention concerne un procédé de production de cellules adhérentes selon lequel après avoir produit des cellules adhérentes dans un premier récipient de culture selon un procédé de l'invention :

a. on transfère la population cellulaire récoltée après avoir soumis la dite population cellulaire à un traitement enzymatique pour détacher les cellules des microporteurs, dans un second récipient de culture dont le volume utile est plus important et qui contient un milieu de culture contenant des microporteurs en quantité plus importante que la quantité de microporteurs qui était présente lors du dernier passage cellulaire effectué dans le premier récipient de culture, et

b. on met en oeuvre l'un des modes de réalisation du procédé de l'invention dans ce second récipient.

Dans un aspect particulier, on répète l'un des modes de réalisation du procédé selon l'invention dans un troisième récipient de culture dont le volume utile est plus important que le volume utile du second récipient de culture.

Il est décrit l'utilisation des cellules qui ont été produites selon le procédé de l'invention pour la production d'agents biologiques.

Il est également décrit un procédé de production de cellules adhérentes dans un récipient de culture qui contient des microporteurs dans un milieu de culture, selon lequel on augmente la quantité de cellules produites d'un facteur $\geq$ 60 en réalisant des passages cellulaires successifs dans un seul et même récipient de culture.

Description détaillée de l'invention

L'invention concerne un procédé de production de cellules adhérentes selon lequel pour amplifier des cellules et constituer des lots industriels de cellules on effectue plusieurs passages cellulaires successifs dans un seul et même récipient de culture cellulaire. Grâce à ce procédé on réduit le nombre de récipients de culture à utiliser et les lots de cellules produits sont homogènes puisqu'ils ont tous le même nombre de passages cellulaires. Ce procédé sert également à la production d'agents biologiques.

Un « passage cellulaire » au sens de la présente invention débute au moment où une suspension de cellules adhérentes sont mises en contact avec des microporteurs dans un milieu de culture et se termine habituellement au moment où les cellules adhérentes sont libérées de leurs microporteurs par traitement enzymatique et se trouvent à nouveau sous la forme d'une suspension dans le milieu de culture. Un passage cellulaire comprend habituellement les phases suivantes :

- une phase de colonisation des microporteurs qui correspond à la période de temps pendant laquelle les cellules qui ont été mises en contact avec les microporteurs dans un milieu de culture adhèrent aux microporteurs ;
- une phase d'amplification des cellules adhérentes aux microporteurs qui correspond à la période de temps pendant laquelle les cellules se multiplient sur les microporteurs jusqu'à ce que la surface disponible des microporteurs colonisés soit recouverte à plus de 70% et de façon préférée à plus de 80% par les cellules. Quand les cellules ont recouvert plus de 70% de la surface disponible des microporteurs colonisés, on considère que les cellules adhérentes sont « substantiellement confluentes » ou ont atteint le « stade de confluence » ; et
- une phase de détachement des cellules substantiellement confluentes des microporteurs au moyen d'un traitement enzymatique de sorte qu'un maximum de cellules soient décrochées de leur support (en général plus de 80% et de façon préférée plus de 90%) dans un espace de temps court (en général en moins de 30 minutes et souvent dans une période de temps inférieure à 20 minutes). La population de cellules est alors essentiellement sous la forme d'une suspension de cellules libérées de leurs microporteurs (ou détachées de leurs microporteurs).

Dans le cas de la présente invention, en fonction de l'utilisation des cellules adhérentes qui sont produites, le dernier passage cellulaire effectué dans le récipient de culture comprend ou ne comprend pas de phase de détachement.

Dans le cadre de la présente invention, les passages cellulaires successifs sont réalisés dans un seul et même récipient de culture en utilisant tout ou partie de la population cellulaire obtenue lors du passage cellulaire précédent pour réaliser le passage cellulaire suivant. On utilise habituellement au moins 80 % de la population cellulaire obtenue lors du passage cellulaire précédent pour réaliser le passage cellulaire suivant. De façon préférée, pour produire une quantité maximale de cellules, on effectue les passages cellulaires successifs en utilisant à chaque fois toute la population cellulaire obtenue lors du passage cellulaire précédent pour réaliser le passage cellulaire suivant. Bien qu'à la fin de chaque passage cellulaire on libère (détache) les cellules de leurs microporteurs au moyen d'un traitement enzymatique, on ne procède pas, comme cela est préconisé dans l'art antérieur, au transfert de la biomasse cellulaire dans un ou plusieurs autres récipients de culture cellulaire pour continuer l'amplification cellulaire. L'amplification de la biomasse cellulaire est réalisée ici dans un seul et même récipient de culture. Ce procédé est très avantageux car on produit dans les mêmes délais des quantités équivalentes de cellules sans avoir à utiliser et à manipuler plusieurs récipients de culture ce qui réduit l'espace nécessaire pour produire des quantités industrielles de cellules et par conséquent réduit sensiblement les couts de production. De façon surprenante, bien que l'amplification de la biomasse cellulaire requière un traitement enzymatique à chaque passage cellulaire, la quantité de cellules qui est produite à la fin de la mise en oeuvre du procédé selon l'invention est significativement plus importante que celle qu'on obtient en utilisant la technologie classique dite de « transfert de microporteur à microporteur » (cf exemple 2).

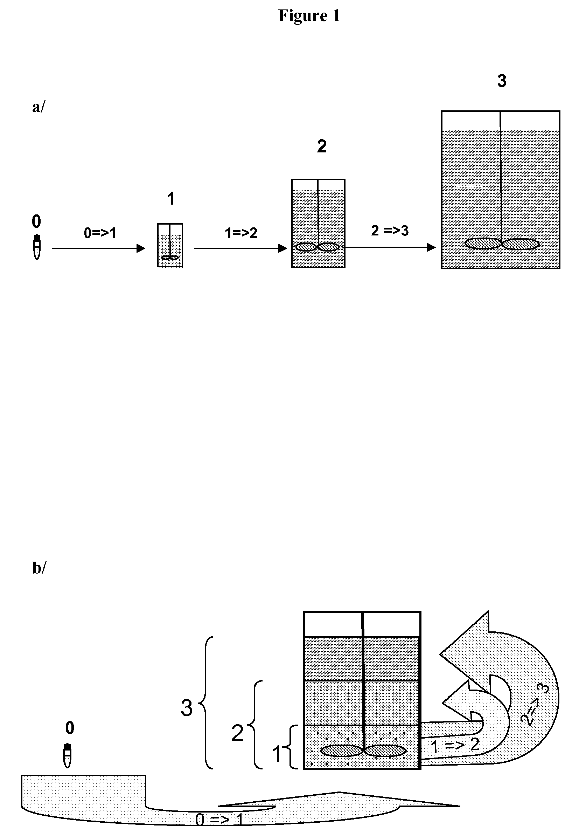

Chaque nouveau passage cellulaire qui correspond à un passage cellulaire ultérieur au premier passage cellulaire est effectué dans le même récipient de culture. Pour démarrer un passage ultérieur au premier passage cellulaire on introduit du milieu de culture et une quantité de microporteurs plus importante que la quantité de microporteurs qui a été introduite lors du passage cellulaire précédent de façon à augmenter la surface du support cellulaire disponible. Il est entendu que le terme de « nouveau passage cellulaire» ou de «passage ultérieur au premier passage cellulaire » désigne un passage cellulaire consécutif à un passage cellulaire qui a été réalisé dans le récipient de culture. Il est également entendu que l'introduction ou l'addition de microporteurs dans le récipient de culture correspond à l'introduction de microporteurs nus. De façon préférée, on utilise des microporteurs non usagés pour faciliter l'adhésion des cellules adhérentes. Même si habituellement on augmente de façon concomitante le volume de milieu de culture à chaque passage ultérieur au premier passage cellulaire, généralement l'augmentation de la quantité de microporteurs est proportionnellement plus importante que l'augmentation de volume de milieu ce qui se traduit généralement par une augmentation progressive de la concentration en microporteurs dans le milieu de culture au cours des passages cellulaires successifs. Pendant le dernier passage cellulaire, lorsque par exemple on utilise comme microporteurs des microbilles de dextran commercialisées sous le nom de cytodex ™ (Cytodex ™ 1, 2 ou 3) la concentration en microporteurs dans le milieu de culture est généralement comprise entre 1 et 7g/l mais peut atteindre 10 à 15g/l. Le procédé de l'invention selon lequel on augmente la biomasse cellulaire par passages cellulaires successifs dans un seul et même récipient de culture est dénommé « procédé all in one » (Voir figure 1b).

Habituellement on effectue 2 passages, 3 passages ou 4 passages cellulaires dans dans le même récipient de culture. En fonction de l'utilisation ultérieure qui en est faite, la population de cellules que l'on récolte à la fin de la mise en oeuvre du procédé selon l'invention est soit, sous la forme d'une suspension de cellules libérées de leur microporteur (dans ce cas, le dernier passage cellulaire est effectué en incluant l'étape de détachement cellulaire) soit, sous la forme d'une suspension de cellules adhérentes aux microporteurs (dans ce cas le dernier passage cellulaire est effectué en omettant l'étape de détachement cellulaire) . Lorsque le procédé de production de cellules adhérentes comprend deux passages cellulaires successifs réalisés dans un seul et même récipient de culture, le procédé selon l'invention revient à mettre à mettre en oeuvre les étapes suivantes:

    a. On introduit du milieu de culture, des microporteurs et des cellules adhérentes dans un récipient de culture, ,
    b. On soumet les cellules à des conditions de culture qui leur permettent d'adhérer et de proliférer sur les microporteurs,
    c. On détache les cellules des microporteurs au moyen d'un traitement enzymatique et on enlève éventuellement une partie des cellules du récipient de culture,
    d. On introduit à nouveau du milieu de culture et des microporteurs , de sorte que la quantité de microporteurs qui est introduite est plus importante que la quantité de microporteurs qui a été précédemment introduite,
    e. On soumet à nouveau les cellules à des conditions de culture qui leur permettent d'adhérer et de proliférer sur les microporteurs, et
    f. On récolte la population cellulaire obtenue après avoir éventuellement détaché les cellules de leurs microporteurs au moyen d'un traitement enzymatique, les étapes a) à e) étant réalisées dans un seul et même récipient de culture.

Les étapes a) à c) correspondent au premier passage cellulaire et les étapes d) à f) correspondent au deuxième passage cellulaire qui se termine par la récolte des cellules.

Lorsqu'il y a plus de deux passages cellulaires successifs réalisés dans le même récipient, cela revient à répéter à nouveau les étapes c), d) et e) après l'étape e) dans le même récipient de culture avant de mettre en oeuvre l'étape f) de récolte des cellules. Habituellement on répète 1 fois les étapes c), d) et e) après l'étape e) ce qui correspond à la réalisation de trois passages cellulaires successifs ou 2 fois les étapes c) d) et e) après l'étape e) ce qui correspond à la réalisation de 4 passages cellulaires successifs. De façon préférée, l'étape c (qui correspond au détachement des cellules de leurs microporteurs) est mis en oeuvre lorsque les cellules sont substantiellement confluentes. Habituellement on augmente aussi le volume de milieu de culture à chaque fois que l'on ajoute des microporteurs (étape d).

Lorsque la population de cellules que l'on récolte à la fin de la mise en oeuvre du procédé selon l'invention sert à constituer un stock de cellules le dernier passage cellulaire comprend généralement une étape de détachement des cellules au moyen d'un traitement enzymatique que l'on effectue généralement dans le même récipient de culture. La population cellulaire est alors essentiellement sous la forme d'une suspension de cellules libérées de leurs microporteurs.

Lorsque la population de cellules sert à la production d'un agent biologique, le dernier passage cellulaire est souvent effectué sans inclure d'étape de détachement cellulaire. La population de cellules produites, sous la forme d'une suspension de cellules adhérentes aux microporteurs, est alors traitée directement dans le même récipient de culture pour qu'elle produise l'agent biologique d'intérêt. Par agent biologique on entend toute substance ou organisme qui peut être produit par les cellules adhérentes. Il s'agit notamment de virus, ou de protéines (anticorps, antigènes enzymes,....). Lorsque le procédé de production d'un agent biologique produit par des cellules adhérentes comprend deux passages cellulaires successifs réalisés dans le même récipient de culture, le procédé selon l'invention équivaut donc à mettre en oeuvre les étapes suivantes :

a. On introduit du milieu de culture, des microporteurs et des cellules adhérentes dans un récipient de culture,
b. On soumet les cellules à des conditions de culture qui leur permettent d'adhérer et de proliférer sur les microporteurs,
c. On détache les cellules des microporteurs au moyen d'un traitement enzymatique et on enlève éventuellement une partie des cellules du récipient de culture,
d. On introduit à nouveau du milieu de culture et des microporteurs, de telle sorte que la quantité de microporteurs nouvellement introduite est plus importante que la quantité de microporteurs qui a été précédemment introduite,
e. On soumet à nouveau les cellules à des conditions de culture qui leur permettent d'adhérer et de proliférer sur les microporteurs,
f. On traite la population cellulaire obtenue de manière à ce qu'elle produise l'agent biologique, et
g. On récolte l'agent biologique, les étapes a), à f) étant réalisées dans un seul et même récipient de culture.

Lorsqu'il y a plus de deux passages cellulaires successifs réalisés dans le même récipient, cela revient à répéter à nouveau les étapes c), d) et e) après l'étape e) dans le même récipient de culture avant de mettre en oeuvre l'étape f) de production de l'agent biologique. Habituellement on répète 1 fois les étapes c), d) et e) après l'étape e) ce qui correspond à la réalisation de trois passages cellulaires successifs ou 2 fois les étapes c) d) et e) après l'étape e) ce qui correspond à la réalisation de 4 passages cellulaires successifs. De façon préférée, l'étape c (qui correspond au détachement des cellules de leurs microporteurs) est mis en oeuvre lorsque les cellules sont substantiellement confluentes. Habituellement on augmente aussi le volume de culture à chaque fois que l'on ajoute des microporteurs (étape d).

Lorsqu'il s'agit de produire une protéine recombinante, comme par exemple une cytokine, un anticorps, une protéine vaccinale, la suspension cellulaire est placée dans des conditions de culture qui favorise la production de cette protéine en utilisant des milieux de production adaptés. On cite à titre d'exemple les milieux décrits dans EP 0354129 pour la production de protéines recombinantes par les cellules CHO et les cellules Vero.

Lorsque l'agent biologique est un agent infectieux on infecte la suspension de cellules adhérentes aux microporteurs en introduisant l'agent infectieux (bactéries, virus, parasites,...) dans le récipient de culture après avoir généralement remplacé le milieu de culture par un milieu d'infection. L'agent biologique infectieux peut être notamment un virus recombinant (poxvirus recombinants, adénovirus recombinants) ou des virus comme par exemple le virus de la rage, de la grippe, de la polio, etc. On récolte habituellement l'agent biologique en prélevant le surnageant de culture en une ou plusieurs fois - voir exemple 7 -. Lorsque l'agent biologique est plutôt intracellulaire comme dans le cas des virus non lytiques, il est souvent avantageux de récolter le surnageant et les cellules que l'on traite ensuite avec des agents lytiques. Les milieux utilisés pour la production d'agents biologiques, notamment les milieux d'infection qui servent à la production de virus comme le virus rabique peuvent être avantageusement dépourvus de sérum d'origine animale, de protéine d'origine animale, ou même de tout produit d'origine animale.

Les microporteurs convenant à l'objet de l'invention sont habituellement sous la forme de microbilles, de préférence non poreuses pour faciliter l'action des enzymes. Elles ont un diamètre généralement compris entre 90 et 250 μm. Leur densité est légèrement supérieure à celle du milieu de culture pour faciliter leur récupération par simple décantation mais en même temps elle ne doit pas être trop élevée pour obtenir une remise en suspension complète des microbilles dès que le milieu est soumis à une agitation modérée. Dans des conditions de culture standard, la densité des microporteurs se situe habituellement entre 1,020 et 1,050 g/ml. La surface des microbilles est choisie pour faciliter l'adhérence des cellules. La matrice des microporteurs est de préférence non rigide pour mieux préserver les cellules lorsque des collisions entre microbilles se produisent. La surface moyenne disponible pour l'adhésion des cellules est habituellement comprise entre 4000 et 5000cm$^2$/g de microbilles. Ces caractéristiques se retrouvent notamment pour des microbilles à matrice de dextran réticulé commercialisées sous le nom de cytodex™ (cytodex 1, cytodex 2, cytodex 3), mais peuvent également se retrouver pour d'autres microbilles dont la matrice est à base de polystyrène réticulé (Biosilon, Solohill) ou pour des microbilles de verre (Sigma Aldrich).

Dans le cadre de la présente invention, la concentration en microporteurs pendant le premier passage cellulaire, en particulier lorsqu'on utilise comme microporteurs des microbilles de cytodex™ tel que des microbilles de cytodex™ 1, est généralement abaissée à une concentration <1g/l alors que dans l'art antérieur les microporteurs sont utilisés à une concentration comprise entre 1 et 5g/l. Elle est habituellement ≤0,5 g/l; Plus spécifiquement elle est comprise entre 0,1 et 0,4 g/l et plus particulièrement elle se situe entre 0,1 et 0,3 g/l. Cette concentration correspond en fait à la concentration initiale en microporteurs dans le milieu de culture après l'introduction des cellules dans le récipient de culture. Elle est donc <1g/l, de façon préférée ≤0,5 g/l ; Elle est en particulier comprise entre 0,1 et 0,4 g/l et plus spécifiquement elle se situe entre 0,1 et 0,3 g/l.

La quantité initiale de cellules qui est introduite dans le récipient de culture est choisie de telle sorte que plus de 80% des microporteurs sont colonisés par les cellules. Pour obtenir ce taux de colonisation, on introduit classiquement dans le récipient de culture une quantité initiale de cellules qui est au minimum 5 à 10 fois plus importante que la quantité de microporteurs qui est présente dans le milieu de culture. Par exemple dans le cas d'une production de cellules Vero, la quantité initiale de cellules qui est introduite dans le récipient de culture est généralement comprise entre 5x10$^3$ et 5 x10$^4$ cellules/cm$^2$ de microbilles de cytodex™ ce qui représente approximativement entre 5 cellules et 50 cellules par microbille. En définitive, puisque la concentration en microporteurs dans le milieu de culture lors du premier passage cellulaire est plus faible que celle qui est classiquement utilisée dans l'état de l'art, il en résulte par conséquent que la concentration cellulaire initiale est également plus faible.

A la fin de chaque passage cellulaire, les cellules sont détachées des microporteurs dans un intervalle de temps court (en général inférieur à 30 minutes et de façon préférée inférieur à 15 minutes) en traitant les cellules avec une solution enzymatique ayant une activité protéolytique (protéase). Dans le cadre de l'invention, on décroche habituellement les cellules des microporteurs dans le récipient de culture qui est utilisé pour effectuer les passages cellulaires successifs ce qui signifie que toutes les phases de culture cellulaire et tous les traitements qui sont effectués sur les cellules au cours des passages successifs sont réalisés dans un seul et même récipient de culture. Il est éventuellement possible de détacher les cellules des microporteurs après les avoir transférées dans un récipient annexe où a lieu le traitement enzymatique puis de réintroduire la suspension de cellules obtenue dans le récipient de culture unique où ont lieu les passages cellulaires successifs. Cette méthode n'est pas avantageuse car elle entraîne une perte en cellules pendant les opérations de transfert.

La solution enzymatique protéolytique renferme habituellement une sérine protéase telle que la trypsine, la pronase® ou la dispase®. On peut utiliser également de la papaïne, de la ficine ou de la collagénase quand les microporteurs sont des microbilles de cytodex 3. Communément, on utilise une solution de trypsine pour détacher les cellules adhérentes des microbilles de cytodex™. De façon préférée, la protéase est d'origine non animale ce qui indique qu'elle a été fabriquée en utilisant un procédé qui n'utilise pas de matière d'origine animale. Elle est fabriquée, par exemple, en utilisant une matière végétale, par synthèse chimique, ou par recombinaison génétique en utilisant des bactéries, des levures, des champignons ou des plantes. On peut par exemple utiliser une solution enzymatique dépourvue de tout produit d'origine animale commercialisée par Invitrogen sous la dénomination commerciale de TrypLE™ Select ou de TrypLE™ Express. Cette protéase dont la séquence protéique est décrite dans WO 94/25583 est produite par fermentation de la souche Fusarium Oxysporum DSM 2672 ou produite par recombinaison génétique. Elle a une activité enzymatique similaire à la trypsine. Pour faciliter le détachement des cellules on peut ajouter à la solution d'enzyme un agent chélatant qui fixe les ions calcium comme par exemple l'EDTA, l'EGTA ou le citrate ou éventuellement on traite les cellules adhérentes avec un agent chélatant avant d'effectuer le traitement enzymatique. La concentration en protéase et éventuellement en agent chélatant dans le milieu ainsi que la température du traitement enzymatique des cellules (habituellement entre 20 et 38°C) sont fixées de telle sorte que plus de 80% des cellules sont détachées de leur support dans un intervalle de temps court (≤30 minutes). Préalablement au traitement enzymatique proprement dit, on retire en général au moins la moitié du volume de milieu de culture. Habituellement on retire environ les 2/3 du volume de milieu de culture. L'activité protéolytique est ensuite neutralisée en ajoutant au milieu un inhibiteur généralement d'origine peptidique ou protéique qui neutralise l'action des protéases. De préférence, la composition de l'inhibiteur est dépourvue de tout contaminant d'origine animale. Il s'agit par exemple de l'aprotinine recombinante, ou d'extraits ou de fractions purifiées contenant un inhibiteur de la trypsine provenant de graines de soja ou de « lima bean » (Worthington Biochemical). Le milieu est généralement maintenu sous agitation pendant toute la phase de détachement des cellules des microporteurs sauf pendant la période ou l'on retire du milieu de culture.

La suspension de cellules obtenue est en général quantifiée à l'aide de systèmes conventionnels de numération qui peuvent déterminer également la viabilité des cellules. Bien que l'on puisse éliminer une partie de la population cellulaire du récipient de culture lorsque la croissance cellulaire a été trop importante, on utilise souvent toute la population cellulaire pour initier un passage cellulaire ultérieur au premier passage cellulaire qui a lieu dans le même récipient. Pour augmenter la biomasse cellulaire au cours des passages successifs, il faut introduire dans le récipient de culture au début de chaque passage ultérieur au premier passage cellulaire (qui débute après l'étape de décrochement des cellules par traitement enzymatique), une quantité de microporteurs qui est plus importante que la quantité de microporteurs qui a été précédemment introduite. Si on maintient le même volume de milieu de culture au cours des passages cellulaires successifs, cela revient à augmenter la concentration en microporteurs à chaque passage cellulaire ultérieur au premier passage cellulaire. Par contre, on peut maintenir la même concentration en microporteurs lors des passages cellulaires successifs si on augmente dans la même proportion le volume de milieu de culture à chaque nouveau passage cellulaire. De façon très préférée, au démarrage de chaque passage cellulaire ultérieur au premier passage cellulaire on augmente à la fois le volume de milieu de culture et la concentration en microporteurs dans le récipient de culture pour amplifier de façon maximale les cellules. A titre indicatif, à chaque nouveau passage cellulaire, les cellules sont cultivées dans un volume de milieu de culture qui est entre 1,2 et 3 fois plus grand que le volume dans lequel se trouvaient les cellules lors du passage précédent. De la même façon, à chaque nouveau passage cellulaire, la concentration en microporteurs dans le milieu de culture est entre 2 et 10 fois plus importante que celle qui existait lors du passage cellulaire précédent. Dans le cadre de la présente invention, il n'est pas utile en général de retirer les microporteurs usagés à la fin de chaque passage cellulaire (c'est-à-dire à la fin de l'étape de décrochement des cellules). Même si ceux-ci peuvent être recolonisés par les cellules, on ne prend généralement pas en compte la quantité de microporteurs usagés provenant des passages cellulaires précédents dans le calcul de la quantité de microporteurs à introduire au début de chaque nouveau passage cellulaire. La phase d'adhésion des cellules aux microporteurs dure généralement entre 1 et 10 heures en fonction du type cellulaire. Après la phase d'adhésion, il peut être avantageux d'éliminer tout ou partie du milieu de culture après avoir laissé décanter les microporteurs et de le remplacer par du milieu neuf pour accélérer la prolifération des cellules adhérentes aux microporteurs.

Les milieux de culture convenant à l'objet de l'invention peuvent être des milieux de culture cellulaire conventionnels supplémentés en sérum d'origine animale. Avantageusement, les milieux de culture contiennent ni sérum, ni protéine sérique. Les milieux de culture peuvent être notamment dépourvus de toute protéine d'origine animale ou même de tout produit d'origine animale. Par « protéine ou produit d'origine animale », on entend une protéine ou un produit dont le procédé de fabrication comprend au moins une étape où on utilise une matière provenant de l'animal ou de l'homme. De façon particulièrement avantageuse, les milieux servant à la culture des cellules peuvent être dépourvus de toute protéine ou contenir de très faibles quantités de protéines sous forme de protéines recombinantes ou extraites de végétaux (soja, riz,...) ou de levures. Ils renferment le plus souvent des protéines de bas poids moléculaire ($\leq$ 10 KD) (encore appelées polypeptides) à des concentrations très faibles. La concentration en protéines totales dans ces milieux de culture est généralement $\leq$ 15 mg/l mesurée par la méthode de Bradford. C'est le cas en particulier du milieu VP SFM commercialisé par InVitrogen qui convient pour le procédé selon l'invention, notamment pour la culture des cellules Vero. On cite également les milieux Opti Pro ™ serum-free (InVitrogen), Episerf (InVitrogen), Ex-cell ® MDCK (Sigma-Aldrich), Ex-Cell ™ Vero (SAFC biosciences) MP-BHK® sérum free (MP Biomedicals), SFC-10 BHK express serum free (Promo cell), SFC-20 BHK express protein free (Promo cell), HyQ PF Vero (Hyclone Réf. SH30352.02), Hyclone SFM4 Megavir , le milieu MDSS2 (Axcell biotechnology), le milieu DMEM Iscove modifié (Hyclone), les milieux nutritifs de Ham (Ham -F10, Ham-F12), le milieu de leibovitz L-15 (Hyclone, le milieu Pro Vero (Lonza) et le milieu Power MDCK (Lonza) qui sont exempts de toute produit d'origine animale et qui contiennent peu ou pas de protéines.

Lorsque le milieu de culture est dépourvu de sérum animal, de protéine sérique ou a une concentration en protéines totales < 15 mg/l (Bradford), on ajoute généralement un agent de protection cellulaire qui protège les cellules contre les forces de cisaillement qui s'exercent lorsque le milieu est agité. Les agents de protection cellulaire les plus fréquemment utilisés ont généralement des propriétés tensioactives. Il s'agit notamment des polymères d'alcool vinylique encore appelés alcools polyvinyliques (PVA), des polymères d'éthylène glycol encore appelés polyéthylène glycols (PEG), des polymères du 1-vinyl- 2-pyrrolidone encore appelés polyvinylpyrrolidone (PVP) ou des poloxamères qui sont des « copolymères en bloc » d'oxyde d'éthylène et d'oxyde de propylène ayant pour formule chimique $HO(C_2H_4O)_a(C_3H_6O)_b(C_2H_4O)_aH$ selon laquelle a désigne le nombre d'unités d'oxyde d'éthylène et b désigne le nombre d'unités d'oxyde de propylène. Ces agents de protection cellulaire sont généralement utilisés dans une gamme de concentration allant de 0,001% à 2% (p/v) dans le milieu de culture. Parmi les agents de protection cellulaire particulièrement préférés on cite le poloxamère 188 et la PVP. Le poloxamère 188 a un poids moléculaire moyen d'environ 8400 daltons et est utilisé dans le milieu de culture à une concentration habituellement comprise entre 0,05 et 0,2% (p/v). La PVP est également recommandée car elle stimule la croissance cellulaire comme cela est décrit dans WO 01/40443. On utilise généralement la PVP dans une gamme de poids moléculaire moyen comprise entre 20 KDa et 360 KDa, de façon préférée dans une gamme de poids moléculaire moyen comprise entre 20 KDa et 40 KDa, à une concentration dans le milieu de culture qui est comprise généralement entre 0,01% et 2% (p/v) et de façon préférée à une concentration comprise entre 0,05% et 0,5% (p/v). La PVP peut être également caractérisée non plus au moyen de son poids moléculaire uniquement mais en fonction de sa valeur K qui prend en compte le poids moléculaire moyen d'une PVP ainsi que les variations de poids moléculaire de part et d'autre de la valeur moyenne. Pour le calcul de la valeur K on se réfère à l'équation telle que définie dans l'article Cryobiology, 8, 453-464 (1971): La valeur K est calculée à partir de la viscosité relative d'une solution à 1% de PVP selon la formule :

$$\mathrm{Log}\ \eta\ \mathrm{rel}/C = 75K_0^2/(1+1.5\ K_0C) + K_0$$

$$K = 1000\ K_0$$

C représente la concentration en grammes de PVP pour 100 ml de milieu.
$\eta$ rel est la viscosité de la solution comparée à celle du solvant.

Une PVP convenant à l'objet de l'invention a une valeur de K qui est généralement comprise entre 18 et 60, de façon préférée entre 26 et 35. A titre indicatif, pour produire un stock de cellules Vero selon le procédé de l'invention, on peut utiliser comme milieu de culture dépourvu de produit d'origine animale et très faiblement dosé en protéines (< 15 mg/l par la méthode de Bradford) un milieu de culture à base de VPSFM commercialisé par Invitrogen contenant comme agent de protection cellulaire une PVP ayant une valeur K autour de 30 à une concentration de 0,1% (p/v) ou du poloxamère 188 à une concentration de 0,1% (p/v).

Habituellement, la composition du milieu de culture est la même au cours des passages cellulaires successifs mais au besoin il peut s'avérer utile d'apporter des suppléments nutritifs tels que du glucose et/ou de la glutamine. Au cours des passages cellulaires successifs, il peut également être utile durant les phases de prolifération des cellules de renouveler tout ou partie du milieu de culture en fonction des besoins des cellules. Ceci est évalué au moyen de méthodes classiques de contrôle à la disposition de l'homme du métier, telles que la mesure du taux de glucose, de glutamine, de lactates, d'ions ammonium. Dans le cadre de la présente invention, le milieu de culture est généralement agité en permanence avec une intensité juste suffisante pour maintenir en permanence les microporteurs en suspension dans le milieu de culture sauf dans les cas où on élimine tout ou partie du milieu de culture.

Le volume du milieu de culture lors du premier passage cellulaire représente habituellement entre la moitié et 1/5 du volume utile du récipient de culture. Dans le cas des récipients de culture de grande capacité (bioréacteurs de plus de 100 litres) dont la configuration particulière permet la culture de cellules adhérentes à des microporteurs dans un petit volume (comme par exemple un bioréacteur équipé d'une zone de sédimentation à fond conique), le volume du milieu de culture peut être plus faible (entre 1/6 et 1/10 du volume utile du bioréacteur) ou même encore plus faible et représenter seulement 1/20, du volume utile du bioréacteur. Comme indiqué précédemment, on augmente généralement les volumes de milieu de culture au cours des passages cellulaires successifs, le dernier passage cellulaire étant souvent réalisé en présence d'un volume de milieu de culture qui correspond à au moins 70% du volume utile du récipient.

Le récipient de culture est équipé d'un système d'agitation (mécanique, par courant d'air,...) permettant de maintenir les microporteurs en suspension dans le milieu de culture cellulaire, et dispose de moyens pour renouveler les milieux en fonction des besoins de la culture et/ou de moyens de contrôle et de régulation de la température, du pH, de la pression en oxygène, du gazage éventuellement en azote ou en air, et des métabolites ou nutriments (lactates, glucose, glutamine, ions ammonium...). Ces dispositifs sont bien connus de l'homme du métier qui sait les mettre en oeuvre en fonction de la taille et la configuration du récipient utilisé. A titre d'exemple, le récipient de culture selon l'invention peut être sous la forme de flacons agités (Spinner), ou de bioréacteur. Lorsque le volume utile du récipient est ≥ 2 litres on utilise habituellement un bioréacteur qui peut être classiquement sous la forme d'une cuve métallique ou d'une cuve de verre réutilisable ou lorsqu'il s'agit de bioréacteurs à usage unique, de récipients sous la forme de poches à usage unique commercialisés notamment par P.Guerin sous la dénomination de Nucleo PG-ATMI™. On peut également utiliser à titre d'exemple le système Biowave (Wave Bioreactor ™) commercialisé par Général Electrics, le système STR diposable Bioreactor ™ (Sartorius), le système SUB ™ (Hyclone), ou le système cell ready (Millipore). Dans le cadre du procédé selon l'invention qui a pour objectif principal de produire des lots de cellules à l'échelle industrielle, on utilise un bioréacteur dont le volume utile est compris entre 3 litres et 1000 litres, mais plus généralement on utilise un bioréacteur dont le volume utile est compris entre 20 litres et 500 litres.

Les « cellules adhérentes » , au sens de l'invention, sont des cellules établies en lignées ou des cellules issues directement de l'extraction de tissus animaux ou humains sains ou tumoraux qui, dans les conditions de culture utilisées, ont besoin d'un support solide pour se multiplier et se développer normalement. Elles forment habituellement une couche unicellulaire sur leur support dû au phénomène d'inhibition de contact. On exclut donc de fait les cellules qui, dans les conditions de culture utilisées, n'ont pas besoin de support solide pour se multiplier et qui poussent en suspension dans le milieu de culture. Les lignées de cellules adhérentes peuvent être issues de cultures primaires de cellules saines ou tumorales mais également peuvent être obtenues par transformation de cellules à l'aide d'agents immortalisants comme c'est le cas de la lignée PER.C6.

Comme exemple de lignées de cellules adhérentes convenant à l'objet de l'invention, on cite les lignées de cellules murines comme la lignée 3T3, NTCT, WEHI, les lignées de cellules de hamster comme la lignée BHK (notamment la lignée BHK21) ou CHO, les lignées de cellules canines comme la lignée MDCK, les lignées de cellules porcines comme la lignée PK15, les lignées de cellules bovines comme la lignée MDBK, les lignées de cellules simiennes comme la lignée Vero, LLC-MK2, FRHL2, MA104, les lignées humaines comme la lignée MRC5, 293, PER.C6, Hela, ECV ou A 431. Ces lignées de cellules adhérentes peuvent être également sous la forme de lignées transfectées avec un vector recombinant (plasmide, virus,...) lorsqu'elles sont destinées à la production de protéines recombinantes.

Grâce au procédé selon l'invention on augmente la population de cellules adhérentes d'un facteur d'au moins 40, de façon préférée d'au moins 60 et de façon particulièrement préférée d'au moins 100 en réalisant des passages cellulaires successifs dans un seul et même récipient de culture. Ceci est réalisable car le procédé selon l'invention permet d'augmenter entre 5 et 40 fois, de façon préférée entre 10 et 30 fois la surface du support cellulaire au cours des passages cellulaires successifs qui sont réalisés dans ce récipient unique. Dans les méthodes de l'art antérieur une amplification cellulaire de cet ordre est observée en utilisant au moins deux récipients, mais plus généralement trois récipients de culture de tailles différentes (voir exemple 5). Le procédé selon l'invention est très avantageux car on produit également dans les mêmes délais les mêmes quantités industrielles de cellules que celles que l'on obtient avec les méthodes de l'art antérieur tout en réduisant les coûts liés à l'utilisation et l'entretien de récipients de culture et l'espace nécessaire pour produire ces lots de cellules.

Le procédé de production de cellules adhérentes selon l'invention peut être avantageusement mis en oeuvre en introduisant directement dans le récipient de culture des cellules qui viennent d'être décongelées sans recourir à une période d'adaptation habituellement préconisée, au cours de laquelle on effectue un ou plusieurs passages cellulaires « d'adaptation » pour « adapter » les cellules à des conditions de culture plus difficiles telles que la culture en présence de microbilles de cytodex™ à une concentration $\leq 0,5$ g/l dans le milieu de culture et/ou la culture dans des milieux qui ne contiennent pas de sérum ou qui contiennent très peu protéines ($\leq 15$ mg/l). Dans le cas d'espèce, on décongèle un stock de cellules adhérentes selon des modalités bien connues de l'homme du métier, puis on introduit directement la suspension de cellules décongelées dans le récipient de culture qui contient des microporteurs dans un milieu de culture pour mettre en oeuvre le procédé tel que décrit dans l'invention. Comme indiqué précédemment, notamment lorsqu'on utilise des microbilles de cytodex™ comme microporteurs, la concentration en microporteurs dans le milieu de culture lors du premier passage cellulaire est abaissée généralement à une concentration $\leq 0,5$ g/l; elle est généralement comprise entre 0,1 et 0,4 g/l et plus spécifiquement elle se situe entre 0,1 et 0,3 g/l. Le milieu de culture n'a pas besoin non plus de contenir de sérum ou de protéines sériques. Le milieu de culture peut même être totalement dépourvu de protéines ou avoir une teneur en protéines totales très faible ($\leq 15$ mg/l). Le stock de cellules congelées peut provenir d'une ampoule (dans ce cas, la quantité de cellules est en générale relativement faible $10^7$ à $5 \times 10^8$ cellules) ou avantageusement provenir d'une poche qui contient jusqu'à 100 fois plus de cellules. On accélère notamment le processus de production à grande échelle de cellules puisqu'on peut ensemencer directement le contenu de poches décongelées dans un récipient de culture de grande contenance.

Lorsque le stock de cellules adhérentes qui a été produit en utilisant le procédé de l'invention n'est pas suffisant, la biomasse cellulaire qui a été obtenue à partir d'un seul et même récipient de culture cellulaire, peut être encore augmentée en transférant la population cellulaire:

o soit dans un ou plusieurs récipients de culture à partir desquels on réalise des passages cellulaires successifs de manière classique, c'est à dire en transférant après chaque passage cellulaire la biomasse cellulaire obtenue dans un autre récipient de culture plus grand; ou plus avantageusement,

o soit dans un second récipient de culture ayant un volume utile beaucoup plus grand (généralement au moins 10 fois plus grand, le plus souvent entre 10 et 50 fois plus grand que le premier récipient) et en appliquant à nouveau le procédé selon l'invention aux cellules qui ont été transférées dans ce second récipient. En opérant ainsi, on réduit encore plus significativement le nombre de récipients de culture à utiliser pour produire des lots industriels de cellules, ainsi que l'espace nécessaire.

Pour évaluer l'intérêt économique de la mise en oeuvre du procédé selon l'invention dans le cadre d'une production de cellules adhérentes à l'échelle industrielle, on peut se référer au schéma classique de production industrielle de cellules Vero destinées à la production du virus polio tel que décrit dans Reviews of Infectious Diseases, vol 6, supplément 2, S341-S344 (1984). Le schéma classique comprend 5 passages cellulaires successifs le premier étant réalisé dans un bioréacteur de 1 litre, le deuxième dans un bioréacteur de 5 litres, le troisième dans un bioréacteur de 20 litres, le quatrième dans un bioréacteur de 150 litres et enfin le cinquième dans un bioréacteur de 1000 litres. Grâce au procédé selon l'invention, on peut effectuer les trois premiers passages cellulaires dans un seul bioréacteur de 20 litres, puis effectuer les deux derniers passages de façon classique en transférant les cellules dans un bioréacteur de 150 litres puis dans un bioréacteur de 1000 litres. On peut également répéter deux fois le procédé selon l'invention en effectuant les trois premiers passages cellulaires dans un seul bioréacteur de 20 litres puis en transférant les cellules obtenues directement dans un seul bioréacteur de 500 ou 1000 litres où on effectue les deux derniers passages cellulaires. Dans les deux cas, on produit dans les mêmes délais une quantité de cellules qui est du même ordre que celle qu'on obtient lorsqu'on applique le schéma classique mais dans le premier cas on fait l'économie de 2 bioréacteurs (1 litre et 5 litres) et dans le deuxième cas une économie de 3 bioréacteurs (1 litre, 5 litres et 150 litres) (cf. exemple 5).

Un procédé de production de cellules adhérentes particulièrement avantageux sur le plan économique consiste à répéter le procédé selon l'invention dans deux récipients de culture de taille très différente. A cet effet, l'invention a donc pour objet :

Un procédé de production de cellules adhérentes selon lequel :

a. on introduit des cellules adhérentes dans un premier récipient de culture qui contient des microporteurs dans un milieu de culture ;

b. on amplifie les cellules en effectuant plusieurs passages cellulaires successifs dans ce premier récipient de culture, dans lequel chaque passage cellulaire ultérieur au premier passage cellulaire est réalisé en utilisant à chaque fois tout ou partie de la population cellulaire qui a été obtenue lors du passage cellulaire précédent et dont les cellules ont été détachées des microporteurs au moyen d'un traitement enzymatique, et en ajoutant à chaque fois du milieu de culture et une quantité croissante de microporteurs;

c. on récolte la population cellulaire obtenue lors du dernier passage cellulaire réalisée dans ce premier récipient de culture après avoir détaché les cellules des microporteurs au moyen d'un traitement enzymatique;

d. on transfère la population cellulaire récoltée dans un deuxième récipient de culture qui a un volume utile plus important et qui contient un milieu de culture contenant des microporteurs en quantité plus importante que la quantité de microporteurs qui était présente pendant le dernier passage cellulaire effectué dans le premier récipient de culture ;

e. on amplifie les cellules en effectuant plusieurs passages cellulaires successifs dans ce deuxième récipient de culture dans lequel chaque passage cellulaire ultérieur au premier passage cellulaire est réalisé en utilisant à chaque fois tout ou partie de la population cellulaire qui a été obtenue lors du passage cellulaire précédent et dont les cellules ont été détachées des microporteurs au moyen d'un traitement enzymatique, et en ajoutant à chaque fois du milieu de culture et une quantité croissante de microporteurs;

f. On récolte la population cellulaire obtenue lors du dernier passage cellulaire réalisée dans ce second récipient de culture après avoir éventuellement détaché les cellules des microporteurs au moyen d'un traitement enzymatique, et éventuellement

g. On répète encore les étapes d à f dans un troisième récipient de culture ayant un volume utile encore plus important.

En général le volume utile du second récipient de culture est 20 à 50 fois plus important que celui du premier récipient de culture.

Avantageusement, les cellules adhérentes qui sont introduites à l'étape a) du procédé proviennent d'un stock de cellules congelées et qui ont été décongelées juste avant l'introduction dans le premier récipient de culture.

La répétition du procédé selon l'invention peut être également mise en oeuvre pour produire un agent biologique. A cet effet, l'invention a donc pour objet : Un procédé de production d'un agent biologique produit par des cellules adhérentes à des microporteurs selon lequel :

a. on introduit des cellules adhérentes dans un premier récipient de culture qui contient des microporteurs dans un milieu de culture ;

b. on amplifie les cellules en effectuant plusieurs passages cellulaires successifs dans ce premier récipient de culture dans lequel chaque passage cellulaire ultérieur au premier passage cellulaire est réalisé en utilisant à chaque fois tout ou partie de la population cellulaire qui a été produite durant le passage cellulaire précédent et dont les cellules ont été détachées des microporteurs au moyen d'un traitement enzymatique, et en ajoutant à chaque fois du milieu de culture et une quantité croissante de microporteurs;

c. On récolte la population cellulaire obtenue lors du dernier passage cellulaire réalisée dans ce premier récipient de culture après avoir détaché les cellules des microporteurs au moyen d'un traitement enzymatique,

d. On transfère la population cellulaire récoltée dans un deuxième récipient de culture qui a un volume utile plus important et qui contient un milieu de culture contenant des microporteurs en quantité plus importante que la quantité de microporteurs qui était présente lors du dernier passage cellulaire effectué dans le premier récipient de culture, ;

e. on amplifie les cellules en effectuant plusieurs passages cellulaires successifs dans ce deuxième récipient de culture dans lequel chaque nouveau passage cellulaire est réalisé en utilisant à chaque fois tout ou partie de la population cellulaire qui a été produite lors du passage cellulaire précédent et dont les cellules ont été détachées des microporteurs au moyen d'un traitement enzymatique, et en ajoutant à chaque fois du milieu de culture et une quantité croissante de microporteurs;

a. On traite la population cellulaire produite lors du dernier passage cellulaire réalisé dans le second récipient de culture pour qu'elle produise l'agent biologique, la dite étape f) étant réalisée dans le même récipient de culture que celui qui a été utilisé pour réaliser les étapes d) et e) et ;

b. on récolte l'agent biologique.

Eventuellement, on peut répéter les étapes d) et e) dans un troisième récipient de culture avant de traiter la population cellulaire pour qu'elle produise l'agent biologique.

Comme indiqué précédemment, l'agent biologique qui est produit peut être par exemple une protéine recombinante ou un virus tel que le virus de la rage.

Il est décrit l'utilisation des cellules qui ont été produites au moyen de l'un des procédés selon l'invention pour la production d'agents biologiques.

Il est également décrit un procédé de production de cellules adhérentes dans un milieu de culture qui contient des microporteurs selon lequel on réalise des passages cellulaires successifs dans un seul et même récipient de culture pour augmenter la population de cellules d'un facteur $\geq 40$, de façon préférée d'un facteur $\geq 60$ et de façon particulièrement préférée d'un facteur $\geq 120$. Le récipient de culture utilisé est de façon préférée un bioréacteur ayant un volume utile d'au moins 20 litres.

Le milieu de culture comme il a été indiqué dans le cadre de l'invention peut être un milieu classique supplémenté en sérum mais de façon préférée le milieu est dépourvu de sérum d'origine animale. De façon particulièrement préférée on utilise un milieu dépourvu de tout produit d'origine animale et dont la concentration en protéines est $\leq 15$ mg/l.

La figure 1 représente deux procédés d'amplification de cellules adhérentes à des microporteurs au moyen de passages cellulaires successifs : a) selon le procédé classique les passages cellulaires successifs sont réalisés dans des récipients de culture différents et de volumes utiles croissants et b) selon le procédé selon

l'invention (procédé all in one), les passages cellulaires successifs sont réalisés dans un seul et même récipient de culture. Les cellules sont sous forme congelées au stade 0. L'étape 0→1 correspond au transfert des cellules décongelées dans un bioréacteur. L'étape 1 correspond au premier passage cellulaire. L'étape 1→2 correspond au transfert de la population cellulaire obtenue à la fin du premier passage cellulaire après traitement avec une enzyme protéolytique pour décrocher les cellules des microporteurs soit dans le cas du procédé a) dans un deuxième bioréacteur ayant un volume utile plus important soit dans le cas du procédé b) dans le même récipient de culture. L'étape 2 correspond au deuxième passage cellulaire. Dans le cas du procédé b) le deuxième passage cellulaire est réalisé généralement dans un volume de milieu de culture plus important et en présence d'une concentration en microporteurs plus importante. L'étape 2→3 correspond au transfert de la population cellulaire obtenue à la fin du deuxième passage cellulaire après traitement avec une enzyme protéolytique pour décrocher les cellules des microporteurs soit dans le cas du procédé a) dans un troisième bioréacteur ayant un volume utile plus important soit dans le cas du procédé b) dans le même récipient de culture. L'étape 3 correspond au troisième passage cellulaire. Dans le cas du procédé b) le troisième passage cellulaire est réalisé généralement dans un volume de milieu de culture plus important et en présence d'une concentration en microporteurs plus importante que lors du second passage cellulaire.

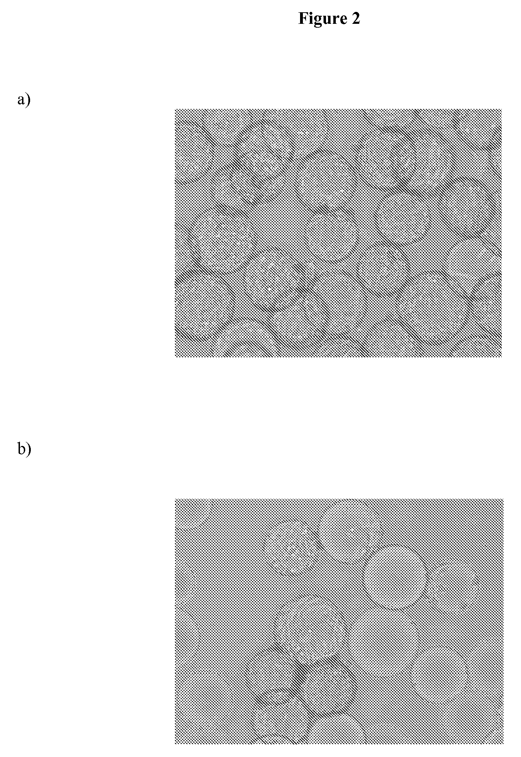

La figure 2 représente l'aspect des microbilles au microscope (grossissement X20) après une période de 8 jours de culture de cellules Vero en mettant en oeuvre a) le procédé « all in one » ou b) le procédé « bead to bead transfer »- (cf exemple 2 pour les conditions opératoires).

La présente invention sera mieux comprise à la lumière des exemples suivants qui servent à illustrer l'invention sans pour autant en limiter le contenu.

Exemple 1 : Amplification des cellules Vero en réalisant 3 passages cellulaires successifs dans un seul et même bioréacteur de 2 litres

Dans cet exemple on a étudié le rôle de différents paramètres tels que la concentration initiale en microporteurs, la présence ou non de sérum dans le milieu de culture ainsi que la nature de l'agent protecteur cellulaire sur la croissance des cellules.

1.1) Matériel utilisé

Bioréacteur :

Les expériences ont été réalisées dans des bioréacteurs à usage unique d'une contenance de 2,4 litres commercialisés sous le nom de Cell ready (Mobius) par Millipore. Ils sont équipés d'une sonde de pH, de $pO_2$ et de température et d'une pâle d'agitation de type hélio marine.

Microporteurs :

On a utilisé des microbilles de cytodex 1 fournies par GE Healthcare. Les microbilles ont été hydratées pendant 24 heures dans un tampon phosphate (PBS 1xC, pH≈7,4) après avoir prélevé la quantité nécessaire pour réaliser chaque passage cellulaire. Elles ont été ensuite rincées 3 fois dans le même tampon puis stérilisés par autoclavage. Juste avant l'introduction dans le bioréacteur on a remplacé le tampon de stérilisation par un volume équivalent de milieu de culture après décantation des microbilles. 1g de microbilles représente une surface d'adhésion d'environ 4400cm$^2$.

Milieux de culture testés

VPSFM/K30 : milieu VPSFM (Invitrogen), sans sérum et sans produit d'origine animale, additionné de 0,1% p/v de polyvinylpyrrolidone (PVP) K30 fourni par ISP VPSFM/F68 : milieu VPSFM (Invitrogen), sans sérum et sans produit d'origine animale, additionné de 0,1% p/v de poloxamère 188 fourni par BASF. VPSFM/K30/SVF : Milieu VPSFM/K30 supplémenté avec 4% de sérum de veau foetal décomplémenté
Cellules :

Cellules Vero provenant d'une banque de cellules conservées sous forme congelée à raison de 50x10$^6$ cellules/ml dans un milieu sans sérum contenant 10% de diméthylsulfoxyde dans des tubes à congélation de cellules (tube Nunc réf. : 430663 de 5ml).

1.2) Protocole opératoire utilisé pour évaluer les paramètres concernant la concentration initiale en microporteurs et l'agent protecteur cellulaire dans le « procédé all in one ».

Le même protocole opératoire a été appliqué pour l'étude de ces deux paramètres.
On a testé les concentrations de 0,1g/l et de 0,3g/l pour évaluer l'effet d'une très faible concentration en microporteurs pendant le premier passage cellulaire sur la croissance des cellules Vero.
Dans le bioréacteur 1 (bio 1) on a introduit 66x10$^6$ cellules dans 2 litres de milieu VPSFM/K30 contenant 0,6g de microporteurs (ce qui équivaut à une quantité de 25 000 cellules/cm$^2$ de surface d'adhésion et représente une concentration initiale en microbilles de 0,3g/l) après avoir ajusté les paramètres de régulation du bioréacteur tel que le pH à 7,2-7,4, la température à 37°C et la pO2 à ≈25%.
Dans le bioréacteur 2 (bio 2) on a introduit 22x10$^6$ cellules dans 2 litres de milieu VPSFM/K30 contenant 0,2g de microporteurs (ce qui équivaut à une quantité de 25 000 cellules/cm$^2$ de surface d'adhésion et représente une concentration initiale en microbilles de 0,1g/l).
Pendant toute la durée de la culture cellulaire le milieu de culture a été soumis à une agitation permanente sauf pendant les phases de décantation des microbilles qui sont utilisées pour renouveler le milieu de culture ou réduire le volume de milieu de culture.
Au jour J3 les cellules ont été traitées à la trypsine selon le protocole suivant : Après décantation des microbilles, on a laissé dans le bioréacteur ≈300 ml de milieu de culture VPSFM/K30 puis on a rajouté ≈ 300 ml d'une solution de citrate de sodium 0,025M contenant 600 mg de trypsine recombinante (réf. : Roche 04618734) en tampon phosphate sans Calcium et sans Magnésium. Le milieu a été maintenu sous agitation modérée. Après avoir vérifié que le décollement des cellules était bien réalisé au moyen d'un prélèvement de contrôle (en général il est réalisé dans un intervalle de 15 et 30 minutes), on a stoppé l'action de la trypsine en rajoutant ≈ 300 ml d'une solution de VPSFM/K30 contenant 1mg/ml d'inhibiteur de trypsine (réf: Sigma T6522). Après numération cellulaire, on a éliminé une partie de la suspension de cellules de sorte que la quantité restante de cellules est telle qu'il y ait ≈ 25x10$^3$ cellules/cm$^2$ de surface d'adhésion après l'introduction de, soit 2,4 g de microporteurs (bio 1), soit 0,6 g de microporteurs (bio 2) dans un volume total de milieu de culture de 2 litres pour réaliser le deuxième passage cellulaire. On a ensuite ajusté à nouveau les paramètres de régulation comme lors du premier passage cellulaire. Au bout de 4 à 6 heures on a remplacé le milieu par du milieu de culture neuf puis éventuellement on a renouvelé une deuxième fois au bout de 24 à 48 heures de culture. A J7, les cellules sont à nouveau trypsinées selon le même protocole que celui utilisé à J3. Après numération cellulaire on a également éliminé une partie de la suspension de cellules de sorte que la quantité restante de cellules est telle qu'il y ait ≈ 25x10$^3$ cellules/cm$^2$ de surface de d'adhésion après introduction de, soit 6 g de microporteurs (bio 1), soit 2,4 g de microporteurs (bio 2) dans un volume total de milieu de culture de 2 litres pour réaliser le troisième passage cellulaire. On a ensuite ajusté à nouveau les paramètres de régulation du bioréacteur. Au bout de 4 à 6 heures on a remplacé le milieu par du milieu de culture neuf puis éventuellement, on a renouvelé une deuxième fois au bout de 24 à 48 heures de culture. A J10, on a récolté les cellules pour évaluer l'intensité de l'amplification cellulaire en fonction de la concentration initiale en microporteurs dans le récipient de culture.
Pour évaluer le rôle des agents protecteurs cellulaires on a testé le poloxamère 188 et la polyvinylpyrrolidone (PVP) K30 en utilisant le même protocole opératoire et avec une concentration initiale en microporteurs de 0,3 g/l.

1.3) Protocole opératoire utilisé pour évaluer le rôle du sérum dans le procédé « all in one ».

Le même protocole opératoire que celui décrit dans le paragraphe précédent a été utilisé avec les spécificités suivantes :

- le milieu VPSFM/K30/SVF a été testé,
- la concentration initiale en microporteurs était de 0,3 g/l.
- les traitements à la trypsine ont été pratiqués aux jours J5 et J8. Avant chaque traitement à la trypsine, on a « rincé » 3 fois la suspension de microbilles avec 600 ml d'un tampon de rinçage (tampon phosphate (PBS 1xC)) au moyen d'une succession d'étapes d'agitation, de décantation et d'élimination du tampon de rinçage pour éliminer le sérum.

1.4) résultats

Pour évaluer le rôle des différents paramètres étudiés sur la croissance cellulaire on a mesuré à intervalles réguliers la concentration cellulaire en utilisant le système de numération Nucleocounter (Chemometec®) et déterminé le nombre de générations cellulaires cumulées. A chaque fois, on a prélevé deux échantillons de la suspension de microbilles. Les valeurs indiquées représentent les valeurs moyennes des deux prélèvements effectués pour chaque temps analysé.

Les quantités de cellules et le nombre de générations cellulaires cumulées observés au cours de la culture sont rassemblés dans les 3 tableaux ci-dessous en fonction de chacun des paramètres étudiés.

1.4.1) paramètre « concentration initiale en microporteurs »

| Jour | Concentration initiale en microporteurs: 0,1g/l | | Concentration initiale en microporteurs : 0,3g/l | |
|------|---------------------------------|------------------------------|------------------------------|---------------------------|
| | Quantité de cellules x $10^6$ | Nombre de générations cumulées*** | Quantité de cellules x$10^6$ | Nombre de générations cumulées |
| J0 | 22 | 0 | 66 | 0 |
| J3* | 159 | 2,85 | 450 | 2,60 |
| J3** | 66 | 2,85 | 264 | 2,60 |
| J4 | 144 | 3,98 | 522 | 3,58 |
| J5 | 267 | 4,87 | 1053 | 4,60 |
| J6 | 690 | 6,24 | 1905 | 5,45 |
| J7* | 822 | 6,47 | 3243 | 6,21 |
| J7** | 375 | 6,47 | 1077 | 6,21 |
| J10 | 2028 | 8,90 | 3150 | 7,76 |

* : avant traitement à la trypsine
** : après traitement à la trypsine et ajustement de la concentration cellulaire à 25x$10^3$ cellules/cm$^2$ de surface d'adhésion.
***: Pour le calcul du nombre de générations cumulées on a pris en compte les éventuels ajustements de la concentration cellulaire qui ont été faits au cours des passages cellulaires successifs et on a utilisé la formule générale ci dessous:

$$\text{Nombre de générations} = \frac{\log_{10}\{[\text{cell}]\text{ finale}/[\text{cell}]\text{ initiale}\}}{\log_{10} 2}$$

[cell] finale : correspond à la concentration cellulaire au jour considéré

[cell] initiale : correspond à la concentration cellulaire initiale à J0

Même si la quantité de cellules produites après 3 passages cellulaires successifs est plus faible dans le bioréacteur où la concentration initiale en microporteurs est de 0,1g/l, le temps de doublement de la population cellulaire est par contre légèrement plus rapide dans ce bioréacteur car le nombre de générations cellulaires cumulées observé est plus élevé pendant toute la durée de l'essai.

De façon surprenante, une concentration très faible en microporteurs pendant le premier passage cellulaire (≈ 0,1g/l) n'a donc pas d'incidence négative sur la croissance des cellules. Au contraire, les cellules ont tendance à se diviser encore plus activement que lorsqu'elles se trouvent dans un bioréacteur qui contient une concentration en microporteurs plus importante (0,3g/l)

1.4.2) paramètre « agent protecteur cellulaire »

| Jour | Agent protecteur : PVP (K30) | | Agent protecteur : Poloxamère 188 | |
|---|---|---|---|---|
| | Quantité de cellules x $10^6$ | Nombre de générations cumulées | Quantité de cellules x$10^6$ | Nombre de générations cumulées |
| J0 | 66 | 0 | 66 | 0 |
| J3* | 450 | 2.60 | 558 | 3.08 |
| J3** | 264 | 2.60 | 264 | 3.08 |
| J4 | 522 | 3.58 | 357 | 3.46 |
| J5 | 1053 | 4.60 | 909 | 4.84 |
| J6 | 1905 | 5.45 | 1215 | 5.26 |
| J7* | 3243 | 6.21 | 2763 | 6.44 |
| J7** | 1077 | 6.21 | 819 | 6.44 |
| J10 | 3150 | 7.76 | 4029 | 8.73 |
| *: avant traitement à la trypsine<br>** : après traitement à la trypsine et ajustement de la concentration cellulaire à 25x$10^3$ cellules/cm$^2$ de surface d'adhésion | | | | |

Les résultats montrent que l'ajout de poloxamère 188 ou de PVP(K30) à un milieu de culture sans sérum ont un effet globalement équivalent sur l'amplification des cellules réalisé selon le procédé all in one.

1.4.3) paramètre « sérum »

| Jour | Milieu de culture avec sérum (VPSFM/K30/SVF) | |
|---|---|---|
| | Quantité de cellules x$10^{6**}$ | Nombre de générations cumulées |
| J0 | 66 | 0 |
| J4 | 993 | 3.91 |
| J5* | 1371 | 4.38 |
| J5** | 264 | 4.38 |
| J6 | 375 | 4.79 |
| J7 | 1098 | 6.40 |
| J8* | 1956 | 7.25 |

(suite)

| Jour | Milieu de culture avec sérum (VPSFM/K30/SVF) | |
|------|----------------------------------------------|-----------------------------------|
| | Quantité de cellules x$10^{6**}$ | Nombre de générations cumulées |
| J8** | 528 | 7.25 |
| J11 | 2289 | 9.33 |
| J12 | 3242 | 9.83 |
| *: avant traitement à la trypsine<br>** : après traitement à la trypsine | | |

Ces résultats montrent que le procédé « all in one » est également applicable à des cellules adhérentes à des microporteures cultivées dans un milieu de culture contenant du sérum.

Exemple 2 : comparaison des productions de cellules Vero obtenues soit en utilisant le procédé selon l'invention (« procédé all in one »), soit en utilisant le procédé par simple ajout de microporteurs (« technique dite de « bead to bead transfer »

On a comparé la production des cellules après 2 passages cellulaires successifs réalisés selon le « procédé all in one » ou en utilisant la technique « bead to bead transfer »

Les études ont été réalisées dans des bioréacteurs en verre, d'une contenance de 4 litres commercialisés par Sartorius sous le nom de Quattro. Ils sont équipés d'une sonde pH, pO2, et de température et d'une pâle d'agitation.

2.1) protocole opératoire utilisé pour la mise en oeuvre du « procédé all in one »

Pour le « procédé all in one », on a appliqué le même principe que celui qui a été décrit dans le paragraphe 1.2 avec les spécificités suivantes :

-   le milieu de culture utilisé était le milieu VPSFM/K30
-   le volume du milieu de culture est resté constant pendant les 2 passages cellulaires successifs et était de 4 litres.
-   Le premier passage cellulaire a été réalisé en utilisant une concentration en microbilles de 0,3 g/l et en introduisant une quantité de cellules de sorte qu'il y ait 50 000 cellules/cm2 de surface d'adhésion.
-   On a renouvelé le milieu de culture à J3
-   A J4 les cellules ont été traitées à la trypsine selon les mêmes modalités opératoires que celles décrites dans le paragraphe 1.2. Après trypsination et numération des cellules, on a éliminé les 2/3 du volume de la suspension cellulaire pour ajuster la quantité restante de cellules à 25 000 cellules/cm2 de surface d'adhésion après introduction de 4,8g de microbilles (soit une concentration de 1,2g/l) dans un volume total de milieu de culture de 4 litres pour réaliser le deuxième passage cellulaire.
-   On a renouvelé le milieu de culture 2 fois : une première fois 4 à 6 heures après l'introduction des microbilles puis une deuxième fois à J6.
-   A J8, les cellules ont été récoltées et comptées pour évaluer l'intensité de l'amplification cellulaire. On a également analysé au microscope une aliquote de la suspension de microbilles pour évaluer le degré de recouvrement des microbilles par les cellules.

2.2) protocole opératoire utilisé pour la mise en oeuvre du procédé « bead to bead transfer »

On appliqué le même protocole opératoire que celui décrit dans le paragraphe 2.1 sauf qu'il n'y a pas eu de traitement à la trypsine des cellules à J4. Au jour J4, on a éliminé les 2/3 du volume de la suspension de microbilles de façon à éliminer la même proportion de cellules et de microbilles pour être dans les mêmes conditions de culture que celles utilisées dans le procédé « all in one ». On a introduit ensuite une suspension de microbilles contenant 4,8g de cytodex 1 (soit une concentration de 1,2g/l) dans un volume total de milieu de culture de 4litres. Le protocole opératoire est ensuite le même que celui qui est décrit dans le paragraphe 2.1.

2.3) Résultats

Pour comparer la croissance cellulaire au cours des deux procédés testés, on a mesuré à intervalles réguliers la concentration cellulaire selon les mêmes modalités que celles décrites au paragraphe 1.4.

Les quantités de cellules et le nombre de générations cellulaires cumulées observés au cours de la culture sont rassemblés dans le tableau ci-après

| Jour | Procédé « all in one » | | Procédé « bead to bead transfer» | |
|---|---|---|---|---|
| | Quantité de cellules x $10^6$ | Nombre de générations cumulées | Quantité de cellules x$10^6$ | Nombre de générations cumulées |
| J0 | 264 | 0 | 272 | 0 |
| J1 | 456 | 0.79 | 416 | 0.61 |
| J2 | 792 | 1.58 | 832 | 1.61 |
| J3* | 1508 | 2.51 | 1536 | 2.50 |
| J3** | 456 | 2.51 | 476 | 2.50 |
| J5 | 1592 | 4.32 | 932 | 3.47 |
| J6 | 2532 | 4.99 | 1236 | 3.87 |
| J7 | 3048 | 5.25 | 1400 | 4.05 |
| J8 | 5420 | 6.09 | 2652 | 4.98 |
| *: avant ajustement de la concentration cellulaire<br>** : après ajustement de la concentration cellulaire et éventuellement traitement à la trypsine (cas du procédé « all in one ») | | | | |

Pendant le premier passage cellulaire (J0 à J3) la croissance des cellules est similaire dans les deux bioréacteurs. Par contre, pendant le second passage cellulaire (J3 à J8), l'amplification cellulaire est beaucoup plus faible dans le bioréacteur où la technologie « bead to bead transfer » a été mise en oeuvre. A J8, la quantité de cellules récoltée est ≈ 2 fois plus faible que celle qui a été récoltée dans le bioréacteur où le procédé « all in one » a été mis en oeuvre. Les résultats concernant l'évolution du nombre de générations cellulaires cumulées vont dans le même sens. A J8, le nombre de générations cellulaires cumulées est >6 pour les cellules qui ont été amplifiées selon le procédé all in one. Il est de 5 pour les cellules qui ont été amplifiées selon la technologie « bead to bead transfer ». Les cellules qui sont amplifiées selon le procédé « all in one » se multiplient donc plus activement. Ces résultats sont surprenants dans la mesure où le procédé « all in one » requiert l'usage de la trypsine, connue pour être préjudiciable à l'intégrité des cellules, pour assurer les passages cellulaires successifs des cellules.

Ces résultats ont été confirmés dans 2 essais indépendants réalisés dans les mêmes conditions opératoires. L'analyse de la variance sur le nombre de générations cellulaires cumulées à J8 fait apparaitre une différence significative (p=0,0137) L'analyse microscopique des microbilles à J8 montre que la très grande majorité des microbilles sont couvertes de cellules lorsque le procédé all in one est mise en oeuvre (voir figure 2a). Par contre, une partie seulement des microbilles sont couvertes de cellules lorsque la technologie « bead to bead transfer » est mis en oeuvre. L'agitation permanente du milieu ou l'agitation intermittente du milieu (en répétant par exemple pendant 2 heures des cycles d'agitation de 5 minutes suivi d'un repos de 20 minutes) après l'addition des microbilles de cytodex 1 à J4 ne modifie pas les résultats de façon sensible (voir figure 2b).

Exemple <u>3</u> : Amplification des cellules Vero en réalisant 2 passages cellulaires successifs dans un seul et même bioréacteur de 20 litres

3.1) Matériel utilisé

Bioréacteur :

On a utilisé un bioréacteur de 20 litres sous la forme d'une poche à usage unique commercialisé par ATMI sous le nom de Nucleo-20. Les sondes de pH, de $pO_2$ et de température après avoir été étalonnées puis stérilisées par autoclavage grâce aux poches « probe holder » ont été installées sur la poche selon le protocole standard ATMI : les connexions Kleenpack® situés sur la poche d'une part et sur le « probe-holder » d'autre part ont été connectées, puis la sonde a été introduite à l'intérieur du bioréacteur à travers la connexion ainsi créée.

**EP 2 516 623 B1**

Microporteurs : microbilles de cytodex 1 fournies par GE Healthcare (cf. paragraphe 1.1)

Milieu de culture : Milieu VPSFM/K30 (cf paragraphe 1.1)

Cellules : Cellules Vero (cf paragraphe 1.1)

3.2) Protocole opératoire

Dans le Nucléo-20, on a introduit 6 litres de milieu de culture VPSFM/K30 puis on a ajouté 1 litre d'une suspension de microbilles contenant 4g de cytodex 1 (ce qui représente une concentration initiale en microbilles de 0,5g/l après l'ajout des cellules). Après avoir ajusté les paramètres de régulation à l'intérieur du nucléo-20, tels que la température à 37°C, le pH à 7,2 -7,4, la $pO_2$ à ≈25%, et soumis le milieu à une agitation modérée pour remettre les microbilles en suspension dans le milieu de culture, on a introduit dans le nucléo $500 \times 10^6$ cellules après décongélation et reprise dans 1 litre de milieu de culture VPSFM/K30. Pendant toute la durée du protocole opératoire le milieu de culture a été soumis à une agitation permanente sauf pendant les phases de décantation des microbilles qui ont lieu pour renouveler le milieu de culture ou réduire le volume de milieu de culture.

Au Jour J2 (2 jours après la mise en culture), on a remplacé le milieu par du milieu VPSFM/K30 neuf.

Au jour J5, les cellules ont été trypsinées selon le protocole suivant :

On a stoppé l'agitation, les régulations de pH et de $pO_2$. Seule la régulation de température a été maintenue. Après décantation des microbilles, on a laissé dans le bioréacteur ≈ 3L de milieu de culture VPSFM/K30 puis on a rajouté ≈ 3 litres d'une solution de citrate de sodium 0,025M contenant 600 mg de trypsine recombinante (réf. : Roche 04618734) en tampon phosphate sans Calcium et sans Magnésium. Le milieu a été ensuite à nouveau agité de façon modérée. Après avoir vérifié que le décollement des cellules était bien réalisé au moyen d'un prélèvement de contrôle (en général il est réalisé dans un intervalle de 15 et 30 minutes), on a stoppé l'action de la trypsine en rajoutant ≈ 3 litres d'une solution de VPSFM/K30 contenant 1mg/ml d'inhibiteur de trypsine (réf: Sigma T6522). On a ensuite rajouté la suspension de microbilles contenant 28 g de cytodex 1, ce qui représente une concentration en microbilles d'environ 1,4g/l dans le milieu après avoir ajusté le volume de milieu de culture à 20 litres avec le milieu de culture VPSFM/K30. On a ensuite ajusté à nouveau les paramètres de régulations à l'intérieur du nucléo-20 comme lors du premier passage cellulaire. Au bout de 4 à 6 heures après l'introduction des microbilles on a remplacé le milieu par du milieu de culture neuf. Un deuxième remplacement du milieu de culture par du milieu de culture neuf a été réalisé à J7. A J9, les cellules étaient substantiellement confluentes. Elles ont été ensuite trypsinées selon le même protocole que celui utilisé à J5. On a déterminé le taux d'amplification cellulaire obtenu après 2 passages cellulaires effectués dans le même bioréacteur à partir de la suspension cellulaire obtenue.

3.3) Résultats

Pour mesurer le taux d'amplification cellulaire obtenu, on a mesuré à intervalles réguliers la concentration cellulaire selon les mêmes modalités que celles décrites dans le paragraphe 1.4.

Les quantités et concentrations cellulaires observées au cours de la culture sont représentées dans le tableau I ci-dessous

| Jour de culture | Volume de culture | Concentration cellulaire dans le milieu (cell/ml) | Concentration cellulaire sur le support (cell/cm2 de microbilles | Quantité de cellules x106 |
|---|---|---|---|---|
| J0 | 8L | 48100 | 21875 | 385*** |
| J1 | 8L | 73500 | 33400 | 588 |
| J2 | 8L | 79500 | 36136 | 636 |
| J5* | 8L | 235000 | 213600 | 3760 |
| J5** | 20L | 145500 | 23600 | 2910 |
| J6 | 20L | 195000 | 31650 | 3900 |
| J7 | 20L | 474000 | 76950 | 9480 |
| J8 | 20L | 807000 | 131000 | 16140 |

(suite)

| Jour de culture | Volume de culture | Concentration cellulaire dans le milieu (cell/ml) | Concentration cellulaire sur le support (cell/cm2 de microbilles | Quantité de cellules x106 |
|---|---|---|---|---|
| J9 | 20L | 1313000 | 213000 | 26260 |

* : avant traitement à la trypsine

** : après traitement à la trypsine et adhésion des cellules

*** : représente la quantité initiale de cellules viables ensemencées

Après deux passages cellulaires successifs réalisés dans un même bioréacteur de 20 litres la population cellulaire a augmenté d'un facteur 68 au bout de 9 jours de culture tandis que la surface du support cellulaire a été augmentée d'un facteur 7.

Exemple 4 : Amplification des cellules Vero en réalisant 3 passages cellulaires successifs dans un seul et même bioréacteur de 20 litres

4.1) Matériel utilisé

Le matériel qui a été utilisé est identique au matériel décrit dans le mode opératoire de l'exemple 3.

4.2) Protocole opératoire

Les passages cellulaires ont été réalisés selon le même protocole que celui décrit dans le paragraphe 3.2 hormis les variations suivantes :

- le premier passage a été réalisé en introduisant $250 \times 10^6$ cellules dans 8 litres de milieu de culture VPSFM/K30 contenant 2 g de microbilles de cytodex 1 (ce qui représente une concentration en microbilles de 0,25 g/l)
- A J5, après la premier traitement à la trypsine, pour réaliser le deuxième passage cellulaire on a rajouté une suspension de microbilles contenant 14 g de cytodex 1, ce qui représente une concentration en microbilles d'environ 1,07g/l après avoir ajusté le volume du milieu de culture à 13 litres avec le milieu de culture VPSFM/K30.
- A J9, après le deuxième traitement à la trypsine, pour réaliser le troisième passage cellulaire on a rajouté une suspension de microbilles contenant 60 g de cytodex 1, ce qui représente une concentration en microbilles d'environ 3g/l après avoir ajusté le volume du milieu de culture à 20 litres avec le milieu de culture VPSFM/K30.

A J12, les cellules étaient substantiellement confluentes. Elles ont été ensuite trypsinées selon le même protocole que celui utilisé à J5.

On a mesuré le taux d'amplification cellulaire obtenu après 3 passages cellulaires effectués dans le même bioréacteur à partir de la suspension cellulaire finale obtenue.

4.3) Résultats

Les quantités et concentrations cellulaires observées au cours de la culture sont représentées dans le tableau ci-après :

| Jour de culture | Volume de culture | Concentration cellulaire dans le milieu ($10^6$ cell./ml) | Concentration cellulaire sur le support (cell/cm2 de microbilles | Quantité de cellules x$10^9$ |
|---|---|---|---|---|
| J0 | 8 | 0.038 | 30230 | 0.26 |
| J1 | 8 | 0.026 | 20684 | 0.18 |
| J2 | 8 | 0.051 | 40572 | 0.36 |
| J5* | 8 | 0.12 | 95465 | 0.84 |
| J5** | 13 | 0.072 | 11688 | 0.93 |
| J6 | 13 | 0.143 | 23214 | 1.85 |

(suite)

| Jour de culture | Volume de culture | Concentration cellulaire dans le milieu ($10^6$ cell./ml) | Concentration cellulaire sur le support (cell/cm2 de microbilles | Quantité de cellules x$10^9$ |
|---|---|---|---|---|
| J7 | 13 | 0.241 | 39123 | 3.13 |
| J8 | 13 | 0.414 | 67207 | 5.38 |
| J9* | 13 | 0.743 | 120616 | 9.65 |
| J9** | 20 | 0.346 | 25064 | 7.72 |
| J10 | 20 | 0.537 | 34870 | 10.74 |
| J11 | 20 | 1.016 | 65974 | 20.32 |
| J12 | 20 | 1.649 | 107077 | 32.98 |
| *: avant traitement à la trypsine<br>**: après traitement à la trypsine et adhésion des cellules | | | | |

Après trois passages cellulaires successifs de cellules Vero réalisés dans un même bioréacteur de 20 litres la population cellulaire a augmenté d'un facteur 126 au bout de 12 jours de culture tandis que la surface du support cellulaire a été augmentée d'un facteur 30.

Exemple 5: Comparaison de la production de cellules Vero en utilisant soit le procédé selon l'invention (les passages cellulaires successifs sont réalisés dans un seul et même bioréacteur) ou soit le procédé classique d'expansion cellulaire (les passages cellulaires successifs sont réalisés à chaque fois dans des bioréacteurs différents et de taille plus importante).

5.1) protocole opératoire

On a étudié et comparé la production de cellules Vero en effectuant soit 3 passages cellulaires successifs dans un seul bioréacteur de 20 litres selon le protocole décrit dans l'exemple 4 et en utilisant une quantité initiale de 250x$10^6$ cellules provenant directement d'une banque de cellules congelées, soit 3 passages cellulaires successifs dans des bioréacteurs en inox différents, le premier dans un bioréacteur de 2 litres, le second dans un bioréacteur de 7 litres et le troisième dans un bioréacteur de 28 litres. Les conditions expérimentales du procédé classique qui ont été utilisées sont les suivantes:

Les cellules Vero après décongélation, ont été tout d'abord adaptées à leurs conditions de culture en effectuant un passage initial dans des Cell Factory (CF10) en introduisant 40x$10^3$ cellules par cm$^2$ de surface d'adhésion dans 2 litres de milieu culture VPSFM/K30. Après environ 5 jours de culture, la population cellulaire obtenue a été récoltée après une étape de trypsination. La population cellulaire récoltée est utilisée pour ensemencer un bioréacteur de 2 litres de volume utile contenant une suspension de 2g de microbilles cytodex 1 dans 2 litres de milieu de culture VPSFM/K30 (concentration 1g/l). Après avoir contrôlé et ajusté les paramètres de régulation tels que la température à 37°C, le pH à 7,2-7,4 et la pO$_2$ à ≈25 %, et soumis le milieu à une agitation modérée, on a ensemencé le bioréacteur de 2 litres avec en moyenne de 220x$10^6$ cellules. A J3, on a remplacé le milieu de culture par du milieu de culture neuf. A J4, les cellules substantiellement confluentes ont été trypsinées puis transférées avec les microporteurs usagés dans un bioréacteur de 7 litres contenant 7 litres de milieu de culture VPSFM/K30 auquel on a préalablement ajouté 14 g de microbilles cytodex 1 ce qui représente une concentration en microbilles d'environ 2g/l. A J6, on a remplacé le milieu de culture par du milieu de culture neuf. A J8, les cellules substantiellement confluentes ont été trypsinées puis transférées de la même façon dans un bioréacteur de 28 litres contenant 28 litres de milieu de culture VPSFM/K30 auquel on a préalablement ajouté 70 g de microbilles cytodex 1 ce qui représente une concentration en microbilles d'environ 2,5g/l. A J10 on a remplacé le milieu de culture par du milieu de culture neuf A J11 les cellules substantiellement confluentes ont été trypsinées puis récoltées et dénombrées.

5.2) Résultats

Pour suivre la croissance cellulaire au cours des deux procédés testés, on a mesuré à intervalles réguliers la concentration cellulaire selon les mêmes modalités que celles décrites dans le paragraphe 1.4.

Les quantités de cellules et les taux d'amplification observés au cours de la culture sont rassemblés dans le tableau ci-dessous

| Jour | Procédé « all in one » 1 seul bioréacteur de 20 litres | | Procédé classique 3 Bioréacteurs (4, 7 et 28 litres) | |
|---|---|---|---|---|
| | Quantité de cellules x 10$^{6*}$ | Taux d'amplification | Quantité de cellules x10$^{6**}$ | Taux d'amplification |
| J0 | 253 | | 220 | |
| J1 | 162 | 0,64 | 446 | 2,03 |
| J2 | 300 | 1,18 | 874 | 3,97 |
| J4 | 832 | 3,29 | 2017 | 9,17 |
| J5 | 1024 | 4,05 | 1499 | 6,59 |
| J6 | 1426 | 5,64 | 2763 | 12,56 |
| J7 | 2448 | 9,68 | 4494 | 20,43 |
| J8 | 4242 | 16,77 | 7251 | 32,96 |
| J10 | 10740 | 42,45 | 16567 | 75,30 |
| J11 | 20320 | 80,32 | 32056 | 145,71 |
| J12 | 29140 | 115,18 | | |
| *: les quantités exprimées sont les valeurs moyennes obtenues sur 8 essais différents qui ont été réalisés. ** : les quantités exprimées sont les valeurs moyennes obtenues sur 3 essais différents qui ont été réalisés. | | | | |

En utilisant le procédé « all in one » on obtient en moyenne plus de 29 Milliards de cellules au bout de 12 jours de culture après avoir introduit en moyenne 253 millions de cellules directement décongelés dans un bioréacteur de 20 litres, soit un taux d'amplification cellulaire moyen de 115. En utilisant le procédé classique, on obtient en moyenne 32 Milliards de cellules au bout de 11 jours de culture après avoir initialement introduit en moyenne 220 millions dans un bioréacteur de 2 litres, soit un taux d'amplification cellulaire moyen de 145. La surface du support cellulaire disponible a été augmentée d'un facteur 30 dans les deux procédés mais a nécessité l'utilisation de 3 bioréacteurs dans le cas du procédé classique. La quantité de cellules obtenue avec le procédé classique est légèrement plus importante. Cela provient du fait que les cellules qui ont été utilisées pour mettre en oeuvre le procédé « all in one » et le procédé classique n'étaient pas dans les mêmes conditions physiologiques. Les cellules qui ont servi à ensemencer le bioréacteur de 20 litres dans le cas du procédé « all in one » venaient juste d'être décongelées tandis que les cellules utilisées pour ensemencer le bioréacteur de 2 litres étaient beaucoup plus vigoureuses puisqu'elles avaient été préalablement cultivées en Cell factory. Les résultats à J1 le montrent très clairement ; dans le procédé all in one, la diminution de près de 40% du nombre de cellules à J1 résulte du phénomène classique de « latence post décongélation ». Dans le même temps les cellules qui avaient été préalablement cultivées en Cell factory se sont multipliées dans le procédé classique (la population cellulaire a doublé). Malgré les conditions initiales de culture nettement défavorables dans le procédé « all in one », on constate qu'à la fin de la culture il y a finalement très peu d'écart entre les quantités de cellules récoltées dans les deux procédés. On conclut que si les conditions initiales de culture avaient été les mêmes dans les deux procédés testés on aurait obtenu les mêmes quantités et les mêmes taux d'amplification de cellules. Le procédé « all in one » est donc très avantageux par rapport au procédé classique puisqu'on produit dans le même délai la même quantité de cellules en faisant des économies de moyens.

Exemple 6: amplification de cellules vero en réalisant 3 passages cellulaires successifs dans un seul et même bioréacteur de 200 litres

6.1) Matériel utilisé

A l'exception du bioréacteur qui est dans le cas présent une poche à usage unique de 200 litres commercialisé par ATMI sous le nom de Nucleo-200, le matériel qui a été utilisé est le même que celui qui est décrit dans l'exemple 3.

6.2) Protocole opératoire

Le protocole opératoire utilisé est similaire à celui qui est décrit dans l'exemple 3 avec les modifications suivantes :

Les cellules Vero après avoir été décongelées ont été préalablement mises en culture dans 2 Cell Factory (CF10) à raison de 40x10³ cellules par cm² de surface d'adhésion et 2 litres de milieu de culture par CF10. Après 5 jours de culture, la population cellulaire a été récoltée après une étape de traitement à la trypsine et a servi ensuite à ensemencer le Nucleo-200.

Le premier passage cellulaire dans le Nucleo-200 a été réalisé en introduisant 2,2x10⁹ cellules dans 50 litres de milieu de culture VPSFM/K30 contenant 25 g de microbilles de cytodex 1 (ce qui représente une concentration en microbilles de 0,5 g/l). Au Jour J3 on a remplacé le milieu de culture par du milieu de culture neuf. Au jour J4, les cellules ont été trypsinées selon le protocole de l'exemple 3 en laissant ≈ 20 litres de milieu dans le Nucleo puis en rajoutant ≈ 20 litres d'une solution de citrate de sodium 0,025M contenant 3000 mg de tryspsine recombinante dans un tampon phosphate contenant ni Calcium ni Magnésium. Après le décollement des cellules, on a stoppé l'action de la trypsine en rajoutant 20 litres d'une solution de VPSFM/K30 contenant 1mg/ml d'inhibiteur de trypsine.

Le second passage cellulaire a été réalisé dans le même nucléo-200 en rajoutant à toute la population cellulaire obtenue une suspension de microbilles contenant 130g de cytodex 1, ce qui représente une concentration en microbilles d'environ 1g/l après avoir ajusté le volume total du milieu à 130 litres avec le milieu de culture VPSFM/K30. On a renouvelé 2 fois le milieu de culture: la première fois juste après l'adhésion des cellules aux microbilles, la seconde fois à J6. Au jour J7, les cellules ont été à nouveau trypsinées selon le même protocole que celui utilisé à J4.

Le troisième passage cellulaire dans le même nucléo- 200 a été réalisé en ajustant la population cellulaire de sorte qu'il y ait une concentration de 20000 cellules/cm² de surface d'adhésion après introduction de 450g de microbilles de cytodex 1 dans un volume total de milieu qui a été ajusté à 180 litres avec le milieu de culture VPSFM/K30. De la même façon, on a renouvelé deux fois le milieu de culture: juste après l'adhésion des cellules aux microbilles, puis à J10. La population de cellules obtenue après les 3 passages cellulaires successifs effectués dans le même bioréacteur a été ensuite quantifiée. On peut mesurer ainsi le taux d'amplification cellulaire.

6.3) Résultats

Les quantités et concentrations cellulaires observées au cours de la culture sont représentées dans le tableau ci-dessous

| Jour de culture | Volume de culture | Concentration cellulaire dans le milieu (10⁶ cell./ml) | Concentration cellulaire sur le support (cell/cm2 de microbilles | Quantité de cellules x10⁹ |
|---|---|---|---|---|
| J0 | 50L | 0.038 | 17580 | 1.93 |
| J1 | 50L | 0.034 | 15455 | 1.71 |
| J2 | 50L | 0.07 | 31818 | 3.50 |
| J3 | 50L | 0.147 | 66818 | 7.35 |
| J4* | 50L | 0.162 | 103000 | 11.34 |
| J4** | 130L | 0.114 | 25900 | 14.81 |
| J5 | 130L | 0.135 | 30600 | 17.55 |
| J6 | 130L | 0.275 | 62500 | 35.75 |
| J7* | 130L | 0.552 | 125455 | 71.76 |
| J7*** | 180L | 0.212 | 19273 | 38.16 |
| J8 | 180L | 0.329 | 29900 | 59.22 |
| J9 | 180L | 0.646 | 58727 | 116.28 |
| J10 | 180L | 1.1 | 100000 | 198 |
| J11 | 180L | 1.5 | 136364 | 270 |
| * : avant traitement à la trypsine | | | | |
| ** : après traitement à la trypsine et adhésion des cellules | | | | |
| *** : après traitement à la trypsine et ajustement de la population cellulaire à la concentration de 20000 cellules/cm² de surface d'adhésion | | | | |

Après trois passages cellulaires successifs de cellules Vero réalisés dans un même bioréacteur de 200 litres la population cellulaire a augmenté d'un facteur 140 au bout de 11 jours de culture tandis que la surface du support cellulaire a été augmentée d'un facteur 18.

Des essais d'amplification de cellules Vero, selon le « procédé all in one » ont également été réalisés en réalisant notamment deux passages cellulaires successifs dans un seul bioréacteur à usage unique de 500 litres commercialisé par ATMI sous le nom de nucleo-500. Les taux d'amplification cellulaire obtenus étaient similaires à ceux que l'on obtient avec les bioréacteurs de 200 litres ce qui montre bien que le procédé de l'invention est exploitable à très grande échelle.

Exemple 7: Production du virus rabique à partir d'un lot de cellules qui a été obtenu en réalisant 3 passages cellulaires successifs dans un seul et même bioréacteur de 200 litres.

Le lot de cellules a été produit en utilisant le même protocole que celui qui a été décrit dans l'exemple 6.

Au jour J11, on a remplacé le milieu de culture par un milieu d'infection virale à base de VPSFM puis on a infecté les cellules avec la souche Pitman Moore du virus rabique provenant de l'institut Wistar à une multiplicité d'infection de 0,01. Les mêmes paramètres de régulation concernant la température, le pH, la pO2 et l'agitation du milieu modérée utilisés pour la culture cellulaire ont été conservés ajustés pour la production de virus. On a renouvelé le milieu d'infection virale au Jour J3 après l'infection virale, puis on a récolté les surnageants de culture pour mesurer les titres infectieux aux jours J7, J10 et J14 après l'infection virale. Après chaque récolte virale, on a remis du milieu d'infection virale neuf Les titres infectieux dans les surnageants de culture ont été mesurés au moyen d'un test classique d'immunofluorescence sur cellules BHK21. On a réalisé une série de dilutions pour chacun des surnageants de culture testés puis chaque dilution a été distribuée dans 10 puits d'une microplaque 96 puits. Deux séries de tests ont été réalisés en parallèle. On a rajouté ensuite une suspension de cellules BHK21 dans chacun des puits. Les cellules ont été incubées pendant 48 heures à 37°C sous 5% de $CO_2$. Au bout de 48 heures les puits étaient recouverts d'un tapis cellulaire que l'on a ensuite fixé à l'acétone. Après avoir éliminé l'acétone et sécher les microplaques, on a rajouté 50 $\mu$l d'une dilution au 1/70ème d'un anticorps monoclonal dirigé contre le virus rabique (FDI Fujirebio Diagnostics-Ref 800092). Après une incubation d'une heure suivie de plusieurs rinçages on a analysé les microplaques au microscope à flurorescence. Un puits est considéré comme positif dès lors que l'on observe une fluorescence spécifique dans au moins une cellule. Comme contrôle négatif on a utilisé les cellules BHK21 cultivées en l'absence de virus rabique et comme contrôle positif, les cellules BHK21 cultivées en présence d'une souche de virus rabique de référence. Les titres infectieux en virus rabiques contenus dans les surnageants de culture testés ont été déterminés selon la méthode de Spearman et Karber et exprimés en unités $\log_{10}$ de dose infectieuse en culture de cellules 50% (DICC 50). Les titres infectieux obtenus dans les surnageants de culture qui ont été observés se situaient aux environs de 7,0 $\log_{10}$ DICC 50.

**Revendications**

**1.** Un procédé de production de cellules adhérentes selon lequel :

    a. on introduit des cellules adhérentes dans un récipient de culture qui contient des microporteurs dans un milieu de culture :
    b. on amplifie les cellules en effectuant plusieurs passages cellulaires successifs dans ce même récipient de culture dans lequel chaque passage cellulaire ultérieur au premier passage cellulaire est réalisé:

        i. en utilisant tout ou partie de la population cellulaire qui a été obtenue lors du passage cellulaire précédent après avoir soumis la population cellulaire à un traitement enzymatique pour détacher les cellules des microporteurs, et
        ii. en introduisant du milieu de culture et une quantité croissante de microporteurs; et

    c. On récolte la population cellulaire produite lors du dernier passage cellulaire après avoir, de façon optionnelle, soumis la population cellulaire à un traitement enzymatique pour détacher les cellules des microporteurs.

**2.** Un procédé de production d'un agent biologique produit par des cellules adhérentes selon lequel :

    a. on introduit des cellules adhérentes dans un récipient de culture qui contient des microporteurs dans un milieu de culture :
    b. on amplifie les cellules en effectuant plusieurs passages cellulaires successifs dans ce même récipient de culture dans lequel chaque passage cellulaire ultérieur au premier passage cellulaire est réalisé :

i. en utilisant tout ou partie de la population cellulaire qui a été obtenue lors du passage cellulaire précédent après avoir soumis la population cellulaire à un traitement enzymatique pour détacher les cellules des microporteurs, et
ii. en introduisant du milieu de culture et une quantité croissante de microporteurs.

c. on traite la population cellulaire produite lors du dernier passage cellulaire de manière à ce qu'elle produise l'agent biologique, le dit traitement étant réalisé dans le même récipient de culture que celui qui a été utilisé pour amplifier les cellules et ;
d. on récolte l'agent biologique.

3. Le procédé selon la revendication 2, selon lequel l'agent biologique est un agent infectieux et en ce que le traitement de la population cellulaire à l'étape c) est effectué en infectant la population cellulaire avec ledit agent infectieux dans un milieu d'infection.

4. Le procédé selon la revendication 3, selon lequel l'agent infectieux est un virus ou un virus recombinant.

5. Le procédé selon la revendication 4, selon lequel le virus est le virus rabique et le milieu d'infection est un milieu d'infection virale dépourvu de tout produit d'origine animale.

6. Le procédé selon l'une des revendications 1 à 5, selon lequel le nombre de passages cellulaires qui sont effectués dans le même récipient de culture est 2, 3 ou 4.

7. Le procédé selon l'une des revendications 1 à 6, selon lequel la concentration en microporteurs dans le milieu de culture pendant le premier passage cellulaire est < 1 g/l, et de façon préférée ≤ 0,5 g/l.

8. Le procédé selon l'une des revendications 1 à 7, selon lequel le traitement enzymatique emploie une solution contenant une enzyme protéolytique.

9. Le procédé selon l'une des revendications 1 à 8, selon lequel chaque passage cellulaire ultérieur est réalisé en augmentant le volume du milieu de culture.

10. Le procédé selon l'une des revendications 1 à 9, selon lequel le premier passage cellulaire est effectué dans un volume de milieu de culture qui est entre le 1/5 et la moitié du volume utile du récipient de culture.

11. Le procédé selon l'une des revendications 1 à 10, selon lequel le milieu de culture est dépourvu de sérum d'origine animale.

12. Le procédé selon l'une des revendications 1 à 11, selon lequel le milieu de culture est dépourvu de tout produit d'origine animale.

13. Le procédé selon l'une des revendications 1 à 12, selon lequel la concentration en protéines dans le milieu de culture est ≤15 mg/l.

14. Le procédé selon l'une des revendications 1 à 13, selon lequel le milieu de culture contient en outre un agent protecteur cellulaire.

15. Le procédé selon la revendication 14, selon lequel l'agent protecteur cellulaire est une polyvinyl pyrrolidone ou un poloxamère.

16. Le procédé selon l'une des revendications 1 à 15, selon lequel le récipient de culture est un bioréacteur ayant un volume utile compris entre 3 et 3000 litres, de façon préférée entre 20 et 1000 litres, et de façon particulièrement préférée entre 20 et 500 litres.

17. Le procédé selon la revendication 16, selon lequel le récipient de culture est un bioréacteur à usage unique.

18. Le procédé selon l'une des revendications 1 à 17, selon lequel les cellules adhérentes sont des cellules Vero.

19. Le procédé selon l'une des revendications 1 et 6 à 18, selon lequel la population cellulaire qui est récoltée à l'étape c) contient une quantité de cellules qui est au moins 60 fois plus importante que la quantité de cellules qui a été initialement introduite à l'étape a) du procédé.

20. Un procédé de production de cellules adhérentes selon lequel :

a. on décongèle un stock de cellules adhérentes, puis
b. on soumet les cellules adhérentes décongelées au procédé tel que revendiqué selon l'une des revendications 1 et 6 à 19.

21. Un procédé de production de cellules adhérentes selon lequel on produit des cellules adhérentes dans un premier récipient de culture selon l'une des revendications 1 et 6 à 20 :

a. on transfère la population cellulaire récoltée après avoir soumis la dite population cellulaire à un traitement enzymatique pour détacher les cellules des microporteurs, dans un second récipient de culture dont le volume utile est plus important et qui contient un milieu de culture contenant des microporteurs en quantité plus importante que la quantité de microporteurs qui était présente lors du dernier passage cellulaire effectué dans le premier récipient de culture, et
b. on met en oeuvre les étapes b) et c) du procédé tel que revendiqué selon l'une des revendications 1 et 6 à 20 dans ce second récipient.

22. Le procédé selon la revendication 21, selon lequel on répète à nouveau les étapes a) et b) de la revendication 21 dans un troisième récipient de culture dont le volume utile est plus important que le volume utile du second récipient de culture.

**Patentansprüche**

1. Verfahren zur Produktion von adhärenten Zellen, bei dem man:

a. adhärente Zellen in ein Kulturbehältnis, das Mikroträger in einem Kulturmedium enthält, einbringt;
b. die Zellen dadurch vermehrt, dass man mehrere aufeinanderfolgende Zellpassagen in demselben Kulturbehältnis durchführt, in dem jede Zellpassage, die sich an die erste Zellpassage anschließt, durchgeführt wird:

i. und zwar unter Verwendung der gesamten oder eines Teils der Zellpopulation, die bei der vorigen Zellpassage, nachdem die Zellpopulation einer Enzymbehandlung zum Ablösen der Zellen von den Mikroträgern unterzogen wurde, erhalten wurde, und
ii. durch Einbringen von Kulturmedium und einer ansteigenden Menge an Mikroträgern; und

c. die in der letzten Zellpassage, nachdem man gegebenenfalls die Zellpopulation einer Enzymbehandlung zum Ablösen der Zellen von den Mikroträgern unterzogen hat, produzierte Zellpopulation erntet.

2. Verfahren zur Produktion eines Biologikums, das von den adhärenten Zellen produziert wird, bei dem man:

a. adhärente Zellen in ein Kulturbehältnis, das Mikroträger in einem Kulturmedium enthält, einbringt;
b. die Zellen dadurch vermehrt, dass man mehrere aufeinanderfolgende Zellpassagen in demselben Kulturbehältnis durchführt, in dem jede Zellpassage, die sich an die erste Zellpassage anschließt, durchgeführt wird:

i. und zwar unter Verwendung der gesamten oder eines Teils der Zellpopulation, die bei der vorigen Zellpassage, nachdem die Zellpopulation einer Enzymbehandlung zum Ablösen der Zellen von den Mikroträgern unterzogen wurde, erhalten wurde, und
ii. durch Einbringen von Kulturmedium und einer ansteigenden Menge an Mikroträgern;

c. die in der letzten Zellpassage produzierte Zellpopulation so behandelt, dass sie das Biologikum produziert, wobei die Behandlung in demselben Kulturbehältnis durchgeführt wird, das für die Vermehrung der Zellen verwendet worden ist;
d. das Biologikum entnimmt.

3. Verfahren nach Anspruch 2, wobei es sich bei dem Biologikum um ein infektiöses Agens handelt und wobei die Behandlung der Zellpopulation in Schritt c) dadurch erfolgt, dass man die Zellpopulation mit dem infektiösen Agens in einem Infektionsmedium infiziert.

4.  Verfahren nach Anspruch 3, wobei es sich bei dem infektiösen Agens um ein Virus oder ein rekombinantes Virus handelt.

5.  Verfahren nach Anspruch 4, wobei es sich bei dem Virus um das Tollwutvirus handelt und bei dem Infektionsmedium um ein Virusinfektionsmedium, das keinerlei Produkt tierischen Ursprungs aufweist, handelt.

6.  Verfahren nach einem der Ansprüche 1 bis 5, wobei die Anzahl der Zellpassagen, die in demselben Kulturbehältnis durchgeführt werden, 2, 3 oder 4 beträgt.

7.  Verfahren nach einem der Ansprüche 1 bis 6, wobei die Mikroträgerkonzentration in dem Kulturmedium während der ersten Zellpassage < 1 g/l, vorzugsweise $\leq$ 0,5 g/l, beträgt.

8.  Verfahren nach einem der Ansprüche 1 bis 7, wobei bei der Enzymbehandlung eine Lösung, die ein proteolytisches Enzym enthält, eingesetzt wird.

9.  Verfahren nach einem der Ansprüche 1 bis 8, wobei jede letzte Zellpassage unter Erhöhung des Kulturmediumvolumens erfolgt.

10.  Verfahren nach einem der Ansprüche 1 bis 9, wobei die erste Zellpassage in einem Kulturmediumvolumen erfolgt, das zwischen 1/5 und der Hälfte des Nutzvolumens des Kulturbehältnisses beträgt.

11.  Verfahren nach einem der Ansprüche 1 bis 10, wobei das Kulturmedium kein Serum tierischen Ursprungs aufweist.

12.  Verfahren nach einem der Ansprüche 1 bis 11, wobei das Kulturmedium keinerlei Produkt tierischen Ursprungs aufweist.

13.  Verfahren nach einem der Ansprüche 1 bis 12, wobei die Proteinkonzentration in dem Kulturmedium $\leq$ 15 mg/l beträgt.

14.  Verfahren nach einem der Ansprüche 1 bis 13, wobei das Kulturmedium weiterhin ein Zellschutzmittel enthält.

15.  Verfahren nach Anspruch 14, wobei das Zellschutzmittel ein Polyvinylpyrrolidon oder ein Poloxamer ist.

16.  Verfahren nach einem der Ansprüche 1 bis 15, wobei es sich bei dem Kulturbehältnis um einen Bioreaktor mit einem Nutzvolumen zwischen 3 und 3000 Litern, vorzugsweise zwischen 20 und 1000 Litern, und besonders bevorzugt zwischen 20 und 500 Litern, handelt.

17.  Verfahren nach Anspruch 16, wobei es sich bei dem Kulturbehältnis um einen Einweg-Bioreaktor handelt.

18.  Verfahren nach einem der Ansprüche 1 bis 17, wobei es sich bei den adhärenten Zellen um Vero-Zellen handelt.

19.  Verfahren nach einem der Ansprüche 1 und 6 bis 18, wobei die Zellpopulation, die in Schritt c) geerntet wird, eine Zellmenge enthält, die mindestens 60-mal größer ist als die Zellmenge, die ursprünglich in Schritt a) des Verfahrens eingebracht wurde.

20.  Verfahren zur Produktion von adhärenten Zellen, bei dem man:

    a. eine Stammkultur von adhärenten Zellen auftaut und anschließend
    b. die aufgetauten adhärenten Zellen dem Verfahren nach einem der Ansprüche 1 und 6 bis 19 unterwirft.

21.  Verfahren zur Produktion von adhärenten Zellen, bei dem man adhärente Zellen in einem ersten Kulturbehältnis nach einem der Ansprüche 1 und 6 bis 20 produziert:

    a. man führt die geerntete Zellpopulation, nachdem man die Zellpopulation einer Enzymbehandlung zum Ablösen der Zellen von den Mikroträgern unterzogen hat, in ein zweites Kulturbehältnis über, dessen Nutzvolumen größer ist und das ein Kulturmedium enthält, das Mikroträger in einer Menge enthält, die größer ist als die Mikroträgermenge, die bei der letzten Zellpassage, die in dem ersten Kulturbehältnis durchgeführt wurde, vorhanden war, und

b. man führt die Schritte b) und c) des Verfahrens nach einem der Ansprüche 1 und 6 bis 20 in diesem zweiten Behältnis durch.

22. Verfahren nach Anspruch 21, wobei man die Schritte a) und b) von Anspruch 21 in einem dritten Kulturbehältnis wiederholt, dessen Nutzvolumen größer ist als das Nutzvolumen des zweiten Kulturbehältnisses.

**Claims**

1. Process for producing adherent cells, according to which:

    a. adherent cells are introduced into a culture vessel which contains microcarriers in a culture medium;
    b. the cells are amplified by performing several successive cell passages in this same culture vessel, wherein each cell passage subsequent to the first cell passage is carried out:

        i. using all or part of the cell population that was obtained during the preceding cell passage, after having subjected the cell population to an enzymatic treatment in order to detach the cells from the microcarriers, and
        ii. by introducing culture medium and an increasing amount of microcarriers; and

    c. the cell population produced during the final cell passage is harvested after having optionally subjected the cell population to an enzymatic treatment in order to detach the cells from the microcarriers.

2. Process for producing a biological agent produced by adherent cells, according to which:

    a. adherent cells are introduced into a culture vessel which contains microcarriers in a culture medium;
    b. the cells are amplified by performing several successive cell passages in this same culture vessel, wherein each cell passage subsequent to the first cell passage is carried out:

        i. using all or part of the cell population that was obtained during the preceding cell passage, after having subjected the cell population to an enzymatic treatment in order to detach the cells from the microcarriers, and
        ii. by introducing culture medium and an increasing amount of microcarriers;

    c. the cell population produced during the final cell passage is treated such that it produces the biological agent, said treatment being carried out in the same culture vessel as that which was used to amplify the cells; and
    d. the biological agent is harvested.

3. Process according to Claim 2, according to which the biological agent is an infectious agent and the treatment of the cell population in step c) is performed by infecting the cell population with said infectious agent in an infection medium.

4. Process according to Claim 3, according to which the infectious agent is a virus or a recombinant virus.

5. Process according to Claim 4, in which the virus is the rabies virus and the infection medium is a viral infection medium free of any product of animal origin.

6. Process according to one of Claims 1 to 5, according to which the number of cell passages which are performed in the same culture vessel is 2, 3 or 4.

7. Process according to one of Claims 1 to 6, according to which the concentration of microcarriers in the culture medium during the first cell passage is < 1 g/l, and preferably $\leq$ 0.5 g/l.

8. Process according to one of Claims 1 to 7, according to which the enzymatic treatment employs a solution containing a proteolytic enzyme.

9. Process according to one of Claims 1 to 8, according to which each subsequent cell passage is carried out while increasing the volume of the culture medium.

10. Process according to one of Claims 1 to 9, according to which the first cell passage is performed in a culture medium volume which is between 1/5 and half the working volume of the culture vessel.

11. Process according to one of Claims 1 to 10, according to which the culture medium is free of serum of animal origin.

12. Process according to one of Claims 1 to 11, according to which the culture medium is free of any product of animal origin.

13. Process according to one of Claims 1 to 12, according to which the protein concentration in the culture medium is ≤15 mg/l.

14. Process according to one of Claims 1 to 13, according to which the culture medium also contains a cell protection agent.

15. Process according to Claim 14, according to which the cell protection agent is a polyvinylpyrrolidone or a poloxamer.

16. Process according to one of Claims 1 to 15, according to which the culture vessel is a bioreactor which has a working volume of between 3 and 3000 liters, preferably of between 20 and 1000 liters, and particularly preferably between 20 and 500 liters.

17. Process according to Claim 16, according to which the culture vessel is a single-use bioreactor.

18. Process according to one of Claims 1 to 17, according to which the adherent cells are Vero cells.

19. Process according to one of Claims 1 and 6 to 18, according to which the cell population which is harvested in step c) contains at least 60 times the amount of cells that were initially introduced in step a) of the process.

20. Process for producing adherent cells, according to which:

   a. a stock of adherent cells is thawed, then
   b. the thawed adherent cells are subjected to the process according to one of Claims 1 and 6 to 19.

21. Process for producing adherent cells, according to which adherent cells are produced in a first culture vessel according to one of Claims 1 and 6 to 20:

   a. the cell population harvested after having subjected said cell population to an enzymatic treatment in order to detach the cells from the microcarriers is transferred into a second culture vessel which has a larger working volume and which contains a culture medium containing a larger amount of micropores than the amount of micropores that was present during the final cell passage performed in the first culture vessel, and
   b. steps b) and c) of the process according to one of Claims 1 and 6 to 20 are implemented in this second vessel.

22. Process according to Claim 21, according to which steps a) and b) of Claim 21 are repeated again in a third culture vessel which has a larger working volume than the working volume of the second culture vessel.

## Figure 1

**Figure 2**

a)

b)

## RÉFÉRENCES CITÉES DANS LA DESCRIPTION

**Documents brevets cités dans la description**

- US 4664912 A
- EP 1060241 A
- EP 0354129 A
- WO 9425583 A
- WO 0140443 A

**Littérature non-brevet citée dans la description**

- **OHLSON.** *Cytotechnology,* 1994, vol. 14, 67-80
- **XIAO et al.** *Chinese Medical Sciences Journal,* 1994, vol. 9, 71-74
- **WANG et al.** *Cytotechnology,* 1994, vol. 31, 221-224
- *Cryobiology,* 1971, vol. 8, 453-464
- *Reviews of Infectious Diseases,* 1984, vol. 6 (2), S341-S344